# EUROPEAN PATENT SPECIFICATION

(11) **EP 2 679 675 B1**
(45) Date of publication and mention of the grant of the patent: **15.03.2017**
(21) Application number: 12749214.8
(22) Date of filing: 24.02.2012
(51) Int. Cl.: C12N 7/00, A01N 63/00, A01P 3/00, C12N 1/14

(54) **MYCOVIRUS, PHYTOPATHOGENIC FUNGUS, PLANT DISEASE CONTROLLING AGENT, METHOD FOR CONTROLLING PLANT DISEASE, AND METHOD FOR ATTENUATING PHYTOPATHOGENIC FUNGUS**
MYKOVIRUS, PHYTOPATHOGENER PILZ, MITTEL ZUR BEKÄMPFUNG VON PFLANZENKRANKHEITEN, VERFAHREN ZUR BEKÄMPFUNG VON PFLANZENKRANKHEITEN UND VERFAHREN ZUR DÄMPFUNG DER WIRKUNG DES PHYTOPATHOGENEN PILZES
MYCOVIRUS, CHAMPIGNON PHYTOPATHOGÈNE, AGENT ET PROCÉDÉ DE LUTTE CONTRE LES MALADIES VÉGÉTALES ET PROCÉDÉ D'ATTÉNUATION D'UN CHAMPIGNON PHYTOPATHOGÈNE

(30) Priority: 24.02.2011 JP 2011038951
(43) Date of publication of application: 01.01.2014
(73) Proprietor: National University Corporation Tokyo University of Agriculture and Technology, Fuchu-shi, Tokyo 183-8538 (JP)
(72) Inventor: MORIYAMA Hiromitsu, Fuchu-shi Tokyo 183-8538 (JP); FUKUHARA Toshiyuki, Fuchu-shi Tokyo 183-8538 (JP); ARIE Tsutomu, Fuchu-shi Tokyo 183-8538 (JP); TERAOKA Tohru, Fuchu-shi Tokyo 183-8538 (JP)
(74) Representative: Denison, Christopher Marcus
(86) International application number: PCT/JP2012/054554
(87) International publication number: WO 2012/115227

(56) References cited:
- WO-A1-2009/093409
- WO-A1-2009/093409
- JP-A- 2001 078 774
- S. URAYAMA ET AL: "Mycoviruses related to chrysovirus affect vegetative growth in the rice blast fungus Magnaporthe oryzae", JOURNAL OF GENERAL VIROLOGY, vol. 91, no. 12, 25 August 2010 (2010-08-25), pages 3085-3094, XP055114420, ISSN: 0022-1317, DOI: 10.1099/vir.0.025411-0
- URAYAMA,S. ET AL.: 'Mycoviruses related to chrysovirus affect vegetative growth in the rice blast fungus Magnaporthe oryzae' J.GEN. VIROL. vol. 91, 2010, pages 3085 - 3094, XP055114420
- HIROMITSU MORIYAMA: 'New biocontroller causing hypovirulence to rice blast fungus' BIOSCIENCE & INDUSTRY vol. 68, no. 5, 01 September 2010, pages 353 - 355, XP008169282
- HIROMITSU MORIYAMA ET AL.: 'Ine Imochibyokin Oyobi Alternaria-kin no Seiiku o Yokusei suru Mycovirus o Sekai de Hajimete Hakken' BRAIN TECHNO NEWS no. 132, 2009, pages 5 - 9, XP008170348

### SEQUENCE LISTING

<110> National University Corporation Tokyo University of Agriculture and Technology
<120> A mycovirus, a plant disease fungus, a control agent for plant disease, a method for controlling plant diseases, and a method for attenuating a plant disease fungus
<130> PH-5109-PCT
<150> JP 2011-038951
   <151> 2011-02-24
<160> 20
<170> PatentIn version 3.5
<210> 1
   <211> 3556
   <212> DNA
   <213> Mycovirus MoCV3
<220>
   <221> CDS
   <222> (121)..(3501)
<400> 1
   gcaaaaaaga gaataaagct ttctcctttt tgcactccat tcaccggtta aaatcgcacg      60
   atcagcccgc acaattaagc cattattaga aaaagctcac tcaacgcaga agcttcgacg     120
   atg gca ggt ggt tat gat gag gat gta ggc tca ctg ggg tca ggt gag       168
   Met Ala Gly Gly Tyr Asp Glu Asp Val Gly Ser Leu Gly Ser Gly Glu
   1               5                   10                  15
   cca gtg ttc ggc gca tca tac acg aac atc tgg cac tca acg ctg ctg       216
   Pro Val Phe Gly Ala Ser Tyr Thr Asn Ile Trp His Ser Thr Leu Leu
               20                  25                  30
   gcg gct gag ggg tca gtg gct cac agt ggg cct gcg ctg tac gca atc       264
   Ala Ala Glu Gly Ser Val Ala His Ser Gly Pro Ala Leu Tyr Ala Ile
           35                  40                  45
   gtg ttg ccc ctc agt tgt ggg aag tcc agc tta gcc tcc gtg ctc agc       312
   Val Leu Pro Leu Ser Cys Gly Lys Ser Ser Leu Ala Ser Val Leu Ser
       50                  55                  60
   ggg tac gat att gat gat atg gtg gtt aac tcg gcg gcc tta cat gcc       360
   Gly Tyr Asp Ile Asp Asp Met Val Val Asn Ser Ala Ala Leu His Ala
   65                  70                  75                  80
   gac gat gaa tgg cgc acg atg ctg gat gcg cga tcg aaa ggt tgg gcg       408
   Asp Asp Glu Trp Arg Thr Met Leu Asp Ala Arg Ser Lys Gly Trp Ala
                   85                  90                  95
   tac gag gac aag gcg gct tac cgg ctg gca aac gac ctg atg ctc cgt       456
   Tyr Glu Asp Lys Ala Ala Tyr Arg Leu Ala Asn Asp Leu Met Leu Arg
               100                 105                 110
   cgg gct agg agg ttc ctc cgc gcg ttc gaa ggc gac gat aac gcc cct       504
   Arg Ala Arg Arg Phe Leu Arg Ala Phe Glu Gly Asp Asp Asn Ala Pro
           115                 120                 125
   gtt gtg tac gtg cac acg cgg gaa ctc gca act gct ttg ggg ctg cgg        552
   Val Val Tyr Val His Thr Arg Glu Leu Ala Thr Ala Leu Gly Leu Arg
       130                 135                 140
   atc ata ttc gat ggg tac gta gag gag gct gcg tgg ctg ggt tgt cgc        600
   Ile Ile Phe Asp Gly Tyr Val Glu Glu Ala Ala Trp Leu Gly Cys Arg
   145                 150                 155                 160
   cgc cag ttg gaa tcc gac gca gtg aca cgc gat cgc gac ttg cga gcg        648
   Arg Gln Leu Glu Ser Asp Ala Val Thr Arg Asp Arg Asp Leu Arg Ala
                   165                 170                 175
   tat cgt ggc cag gta gct gcc aac cgg gcg cac gcg ata cgc cac agg        696
   Tyr Arg Gly Gln Val Ala Ala Asn Arg Ala His Ala Ile Arg His Arg
               180                 185                 190
   caa cct gag cca gtc ccg tac aca tcg cac tcc cgg ttg gct gag gcg        744
   Gln Pro Glu Pro Val Pro Tyr Thr Ser His Ser Arg Leu Ala Glu Ala
           195                 200                 205
   gcc gaa gcc gct atc acc cgc gct ggc cta tgc gct ggg agc ccg cgt        792
   Ala Glu Ala Ala Ile Thr Arg Ala Gly Leu Cys Ala Gly Ser Pro Arg
       210                 215                 220
   gac ttg gcg gac agg act aag tta tgc ggg gca cca ccg caa att atg        840
   Asp Leu Ala Asp Arg Thr Lys Leu Cys Gly Ala Pro Pro Gln Ile Met
   225                 230                 235                 240
   ctt gac ttg gcg cac tcg atc tgc cgt gat cgc cac cgg cca gcc tgg        888
   Leu Asp Leu Ala His Ser Ile Cys Arg Asp Arg His Arg Pro Ala Trp
                   245                 250                 255
   ttg cgg gcg gtg gct gcc aag ctg ctg cga tac cgg atg ggc gag gtg        936
   Leu Arg Ala Val Ala Ala Lys Leu Leu Arg Tyr Arg Met Gly Glu Val
               260                 265                 270
   ttg cca cag gag gcg ctg gcg gcc gac aac tat agt gag tgg gcg agg        984
   Leu Pro Gln Glu Ala Leu Ala Ala Asp Asn Tyr Ser Glu Trp Ala Arg
           275                 280                 285
   gtg atc cac gcg act gac cag cac cgt gtg gct gag gct ccc gca caa       1032
   Val Ile His Ala Thr Asp Gln His Arg Val Ala Glu Ala Pro Ala Gln
       290                 295                 300
   tcg ctg aga ggg cag aac tgg tca gag gtt ttt ccg tat ggt gcg ggc       1080
   Ser Leu Arg Gly Gln Asn Trp Ser Glu Val Phe Pro Tyr Gly Ala Gly
   305                 310                 315                 320
   aac tcg cga ttt gct tta gtt aag atc ggt gat tgg atc gat tgc aca       1128
   Asn Ser Arg Phe Ala Leu Val Lys Ile Gly Asp Trp Ile Asp Cys Thr
                   325                 330                 335
   ggc acg agt gcg atg ggc ttc ggt tac gag tgg ttc cgc cag atg gtg       1176
   Gly Thr Ser Ala Met Gly Phe Gly Tyr Glu Trp Phe Arg Gln Met Val
               340                 345                 350
   acc aga cga gag ggc acg tat gaa cag gcg agc tgc atg ctg ctg atg       1224
   Thr Arg Arg Glu Gly Thr Tyr Glu Gln Ala Ser Cys Met Leu Leu Met
           355                 360                 365
   ggc gat gtg ttc gac tac atg gcc ccc gag ttg cac ccg ctg atc caa       1272
   Gly Asp Val Phe Asp Tyr Met Ala Pro Glu Leu His Pro Leu Ile Gln
       370                 375                 380
   cgc tta ccc atg ggg tca ctg agg ttg gag cat tat gct gag atc gcg       1320
   Arg Leu Pro Met Gly Ser Leu Arg Leu Glu His Tyr Ala Glu Ile Ala
   385                 390                 395                 400
   aag gag att cac agg cta gtg cgc tcg agc gta acg ttg ctg ggc cgc       1368
   Lys Glu Ile His Arg Leu Val Arg Ser Ser Val Thr Leu Leu Gly Arg
                   405                 410                 415
   cgc ctg gac gca ggc cag ctc tcc gtg tgc acc tac tgg gat tgc tta       1416
   Arg Leu Asp Ala Gly Gln Leu Ser Val Cys Thr Tyr Trp Asp Cys Leu
               420                 425                 430
   gcg ggg cgc tat ttg ggc tcg ggc gac atg gag aag gag ctg gct gac       1464
   Ala Gly Arg Tyr Leu Gly Ser Gly Asp Met Glu Lys Glu Leu Ala Asp
           435                 440                 445
   cgg acc agc gag cag aaa ccg cgc gtg tgg gtg tca cgg gat ggg acg       1512
   Arg Thr Ser Glu Gln Lys Pro Arg Val Trp Val Ser Arg Asp Gly Thr
       450                 455                 460
   cag tca gcc gac cgg ttc gcg cat gag ttc gct tgt gag gtc agg gct       1560
   Gln Ser Ala Asp Arg Phe Ala His Glu Phe Ala Cys Glu Val Arg Ala
   465                 470                 475                 480
   tta ctg cac caa aca ata gct gac ggt ggt gag caa atg cgc acc gtc       1608
   Leu Leu His Gln Thr Ile Ala Asp Gly Gly Glu Gln Met Arg Thr Val
                   485                 490                 495
   acg gac atg gtg gcg tca ttc gac acg ttc cta gag tat cgc aaa aag       1656
   Thr Asp Met Val Ala Ser Phe Asp Thr Phe Leu Glu Tyr Arg Lys Lys
               500                 505                 510
   tgg gtg aga ccg ggg tcc gta aca ggc agc ccc aag gcc gat ata tat       1704
   Trp Val Arg Pro Gly Ser Val Thr Gly Ser Pro Lys Ala Asp Ile Tyr
           515                 520                 525
   ttg gag gcc gtg agc gag aga gag ggg atg att gcc gaa gtc gct gac       1752
   Leu Glu Ala Val Ser Glu Arg Glu Gly Met Ile Ala Glu Val Ala Asp
       530                 535                 540
   gac att gcg gcg atg ggc acg tac gta ctg gct aac gtc cgg ctg aat       1800
   Asp Ile Ala Ala Met Gly Thr Tyr Val Leu Ala Asn Val Arg Leu Asn
   545                 550                 555                 560
   aag gct gcc acg ttt gag ttc gct gaa ttc ccg gcc ata gta aag cgc       1848
   Lys Ala Ala Thr Phe Glu Phe Ala Glu Phe Pro Ala Ile Val Lys Arg
                   565                 570                 575
   gtg ctg gcg gac tat gtg ccg aac agc ttc aca agg tac ttc ata aag       1896
   Val Leu Ala Asp Tyr Val Pro Asn Ser Phe Thr Arg Tyr Phe Ile Lys
               580                 585                 590
   aac gag att ggc aag cca gcg ggc cga gcg ctt tac ccg tcc cac ctg       1944
   Asn Glu Ile Gly Lys Pro Ala Gly Arg Ala Leu Tyr Pro Ser His Leu
           595                 600                 605
   gcg cat tac gta gcg ggg cag ttc gca ctg tac gcc ctg atg aag gcg       1992
   Ala His Tyr Val Ala Gly Gln Phe Ala Leu Tyr Ala Leu Met Lys Ala
       610                 615                 620
   caa cct atc cct aag gtg cgc ttg gcc tca gaa cgc gac gtg gct atg      2040
   Gln Pro Ile Pro Lys Val Arg Leu Ala Ser Glu Arg Asp Val Ala Met
   625                 630                 635                 640
   gat gaa cac tgg atg tgg atg cag gcc cgt gag ttc acc gtg ggt gtg      2088
   Asp Glu His Trp Met Trp Met Gln Ala Arg Glu Phe Thr Val Gly Val
                   645                 650                 655
   atg ctg gat tac gac aac ttc aac gaa aag cat gaa ttc gcg gat atg      2136
   Met Leu Asp Tyr Asp Asn Phe Asn Glu Lys His Glu Phe Ala Asp Met
               660                 665                 670
   caa ctt atc atg cgg gag ctg aaa gga ttg tat cgc aca gcg ggc gta      2184
   Gln Leu Ile Met Arg Glu Leu Lys Gly Leu Tyr Arg Thr Ala Gly Val
           675                 680                 685
   ttg agc ccc gat tta aag acc atg ata gat tgg gtg gcg gag gcg tat      2232
   Leu Ser Pro Asp Leu Lys Thr Met Ile Asp Trp Val Ala Glu Ala Tyr
       690                 695                 700
   gac cgc acg gtt ttg gag tac gat ggc gaa ctg cac agt ttc aag cac      2280
   Asp Arg Thr Val Leu Glu Tyr Asp Gly Glu Leu His Ser Phe Lys His
   705                 710                 715                 720
   ggc atg cta tcc ggg cag gcg cct acc tcc gca ata aac aac ata ata      2328
   Gly Met Leu Ser Gly Gln Ala Pro Thr Ser Ala Ile Asn Asn Ile Ile
                   725                 730                 735
   aac ggt gcg aac aaa agg ttg ttg ata agg cag gtg gag gag cta aca      2376
   Asn Gly Ala Asn Lys Arg Leu Leu Ile Arg Gln Val Glu Glu Leu Thr
               740                 745                 750
   ggc cgg gtg ata ttc cag aag cgc acg tct ggc ggc gac gac gtg gcc      2424
   Gly Arg Val Ile Phe Gln Lys Arg Thr Ser Gly Gly Asp Asp Val Ala
           755                 760                 765
   ggc gag acc tac tca ctg tac gac gca tac cta gct gtg aag tgc ggg      2472
   Gly Glu Thr Tyr Ser Leu Tyr Asp Ala Tyr Leu Ala Val Lys Cys Gly
       770                 775                 780
   cag cag atg ggg ctg gcc ttc aag gat ata aaa cag ctc tta agc tcc      2520
   Gln Gln Met Gly Leu Ala Phe Lys Asp Ile Lys Gln Leu Leu Ser Ser
   785                 790                 795                 800
   gat tac tat gaa ttt ttt cgg cta ttt gtg agt gtg aag ggc gtg cac      2568
   Asp Tyr Tyr Glu Phe Phe Arg Leu Phe Val Ser Val Lys Gly Val His
                   805                 810                 815
   ggg tcg ctg cct cgt gcg ttg ggt agc atc tgc tca ggc cag tgg tcg      2616
   Gly Ser Leu Pro Arg Ala Leu Gly Ser Ile Cys Ser Gly Gln Trp Ser
               820                 825                 830
   aac agc gtc aaa gcc aaa ttc gtc gac ccg gcg gcc aag cta tcg tcc      2664
   Asn Ser Val Lys Ala Lys Phe Val Asp Pro Ala Ala Lys Leu Ser Ser
           835                 840                 845
   gtg act gat gcg gcc ttc aag ata gct cgc cgg gcg ggg ggc aac gcc      2712
   Val Thr Asp Ala Ala Phe Lys Ile Ala Arg Arg Ala Gly Gly Asn Ala
       850                 855                 860
   aca ttc cgc gag aag cta tgc gcc aca gcg ttc aag aaa tgg gct tca      2760
   Thr Phe Arg Glu Lys Leu Cys Ala Thr Ala Phe Lys Lys Trp Ala Ser
   865                 870                 875                 880
   tac aac gag cag gcg cta gtc agg ggg ttt atc cac ggt gaa agg cac      2808
   Tyr Asn Glu Gln Ala Leu Val Arg Gly Phe Ile His Gly Glu Arg His
                   885                 890                 895
   tcc ggc ggc ctc ggt gtc ccg atg agt gac ggc tcc gtg cta cgc ata      2856
   Ser Gly Gly Leu Gly Val Pro Met Ser Asp Gly Ser Val Leu Arg Ile
               900                 905                 910
   gag cca atc cag tgg cca gat gag gag agg gtg cgt ctc aaa ggc ttg      2904
   Glu Pro Ile Gln Trp Pro Asp Glu Glu Arg Val Arg Leu Lys Gly Leu
           915                 920                 925
   cct aag gac gcg agc caa gtg gtg gtg gaa gat gcc gtg aaa caa gcg      2952
   Pro Lys Asp Ala Ser Gln Val Val Val Glu Asp Ala Val Lys Gln Ala
       930                 935                 940
   acc gag tta gta ggg cct gat agc gtg gag tcg gcc gaa gtt gtt gcc      3000
   Thr Glu Leu Val Gly Pro Asp Ser Val Glu Ser Ala Glu Val Val Ala
   945                 950                 955                 960
   aac cgg ctg agt gag cag gtg ttc aaa gcg aac gtg gca gcg atg gaa      3048
   Asn Arg Leu Ser Glu Gln Val Phe Lys Ala Asn Val Ala Ala Met Glu
                   965                 970                 975
   ggg tcg cgg gtc ggt cag cta ctc gga tca tgg gag ggg ccg cgt cat      3096
   Gly Ser Arg Val Gly Gln Leu Leu Gly Ser Trp Glu Gly Pro Arg His
               980                 985                 990
   gtc agg gtg cac gaa gtg ctg cgt  atc tcg gag gcc gac  gtg gcc gca    3144
   Val Arg Val His Glu Val Leu Arg  Ile Ser Glu Ala Asp  Val Ala Ala
           995                 1000                1005
   acg gcg  ccc acc gca gaa gaa  ttc agg gcg gcg tac  gcc aag cac      3189
   Thr Ala  Pro Thr Ala Glu Glu  Phe Arg Ala Ala Tyr  Ala Lys His
       1010                 1015                 1020
   aag acg  ata ata gaa tac tac  cgc aaa gca ggg gct  aag tat gat      3234
   Lys Thr  Ile Ile Glu Tyr Tyr  Arg Lys Ala Gly Ala  Lys Tyr Asp
       1025                 1030                 1035
   gcg tta  gcc ggg gta gtc aag  ccc aag gcg cgt gaa  aag ctg gcg      3279
   Ala Leu  Ala Gly Val Val Lys  Pro Lys Ala Arg Glu  Lys Leu Ala
       1040                 1045                 1050
   cgc gcg  tca tgc aac ggc acg  ccg tgt gac tac aag  aaa ctg tac      3324
   Arg Ala  Ser Cys Asn Gly Thr  Pro Cys Asp Tyr Lys  Lys Leu Tyr
       1055                 1060                 1065
   ttc tgg  aaa gag cat tta aca  atg tac ggc tgt gga  acg tac ctg      3369
   Phe Trp  Lys Glu His Leu Thr  Met Tyr Gly Cys Gly  Thr Tyr Leu
       1070                 1075                 1080
   ctg act  gaa gac act tac gat  gcg gca agt atg ctg  gcg cta gtg      3414
   Leu Thr  Glu Asp Thr Tyr Asp  Ala Ala Ser Met Leu  Ala Leu Val
       1085                 1090                 1095
   gtg agt  tca gag ctg tcg aat  gaa gcg gtg agt cgt  cgg ttg gct      3459
   Val Ser  Ser Glu Leu Ser Asn  Glu Ala Val Ser Arg  Arg Leu Ala
       1100                 1105                 1110
   gaa tgc  gcg gtc gca ctg aag  cgc gcc ggg ctg gtg  agc tac          3501
   Glu Cys  Ala Val Ala Leu Lys  Arg Ala Gly Leu Val  Ser Tyr
   1115 1120 1125
   tgatcgggca ggtggcacac agcacaaaaa cttcttacgc tgtgaggtgt aagta 3556
<210> 2
   <211> 3252
   <212> DNA
   <213> Mycovirus MoCV3
<220>
   <221> CDS
   <222> (341)..(3142)
<400> 2
   aaaaaagaga ataaagcatt cccttttttg cactttgcgc gacctactca gatggcagct      60
   caaccgaacc gtggcttcga ctcattcttc gcagattact acactcaaga cggcttttcg     120
   gacctaacca gctcacggcc atctaccatc cgcccagcca acatagtggt ggaaccagac     180
   caggaagtct tcgacgccta ccgcgcacac ggcgggagcc ctccggacta cacaaccagt     240
   caagtagctg acgcgttcgg ccagcgtgct tcggcgagta acagaatcca cggagtcagg     300
   cgagacacag ccgcctctgt acacctcgca ggacgaagcg atg ttg atg cgc gag       355
                                               Met Leu Met Arg Glu
                                               1               5
   agc agg ggt atg gaa gag gag gtt gcc agc agc cgc gag gcg ctg cag       403
   Ser Arg Gly Met Glu Glu Glu Val Ala Ser Ser Arg Glu Ala Leu Gln
                   10                  15                  20
   ggc tgg gga gcg tcg ccg gca gcc ggc tgg ccg aac cgc gaa acg aat       451
   Gly Trp Gly Ala Ser Pro Ala Ala Gly Trp Pro Asn Arg Glu Thr Asn
               25                  30                  35
   gga tac gcc caa gac agg agg cct ggg gca caa gcg ccg ctt acg caa       499
   Gly Tyr Ala Gln Asp Arg Arg Pro Gly Ala Gln Ala Pro Leu Thr Gln
           40                  45                  50
   gag agc gca tgg gac ctc gga tcg tgg acg tcc cat aca gag cac caa       547
   Glu Ser Ala Trp Asp Leu Gly Ser Trp Thr Ser His Thr Glu His Gln
       55                  60                  65
   ccg tcg atg agt cgg aac gcg atg ctt ggg agt gcc gtt ggt gcg ggg       595
   Pro Ser Met Ser Arg Asn Ala Met Leu Gly Ser Ala Val Gly Ala Gly
   70                  75                  80                  85
   cgg agt act gga acc aac gaa gcg cgg agg agc acg gcc gag cct gca       643
   Arg Ser Thr Gly Thr Asn Glu Ala Arg Arg Ser Thr Ala Glu Pro Ala
                   90                  95                  100
   gca ggc gcc cgc gca cac ggc ccg agc cag cct ggg aac ctc cta ccc       691
   Ala Gly Ala Arg Ala His Gly Pro Ser Gln Pro Gly Asn Leu Leu Pro
               105                 110                 115
   agc acc agc gta tac agg agt ggg cgg atg gag cag cct acc caa tcc       739
   Ser Thr Ser Val Tyr Arg Ser Gly Arg Met Glu Gln Pro Thr Gln Ser
           120                 125                 130
   agg cgc tac ccg gcg ccg ttg cga acc aag ccc acg aag agc caa cct       787
   Arg Arg Tyr Pro Ala Pro Leu Arg Thr Lys Pro Thr Lys Ser Gln Pro
       135                 140                 145
   cac gtg gaa cag gcg ggc ggg tat acc agg cag cgt gcg acg gcg acg       835
   His Val Glu Gln Ala Gly Gly Tyr Thr Arg Gln Arg Ala Thr Ala Thr
   150                 155                 160                 165
   gac gtg ttg agc gac gca gga ggc ggc cat cca acg caa cga cca ctg       883
   Asp Val Leu Ser Asp Ala Gly Gly Gly His Pro Thr Gln Arg Pro Leu
                   170                 175                 180
   cca cga cgc gga gct ggg ttc gac aca gcg atg ggt cac tca ccg tac       931
   Pro Arg Arg Gly Ala Gly Phe Asp Thr Ala Met Gly His Ser Pro Tyr
               185                 190                 195
   gaa ccg agc gcc gaa tta ccg aca gag agc cag caa tcg tac gcg cta       979
   Glu Pro Ser Ala Glu Leu Pro Thr Glu Ser Gln Gln Ser Tyr Ala Leu
           200                 205                 210
   tcg ccc ggt tca ttt cag gat gat gcc ttg gct gcc cta tcg aca ctg      1027
   Ser Pro Gly Ser Phe Gln Asp Asp Ala Leu Ala Ala Leu Ser Thr Leu
       215                 220                 225
   ggt aag gtg ccg gca ttg gag gtg gcg gga ata gta cgg cga ggg gcc      1075
   Gly Lys Val Pro Ala Leu Glu Val Ala Gly Ile Val Arg Arg Gly Ala
   230                 235                 240                 245
   act ata ctg gga aag ctc gag ccg ccg agc gag gag cag acg tac gct      1123
   Thr Ile Leu Gly Lys Leu Glu Pro Pro Ser Glu Glu Gln Thr Tyr Ala
                   250                 255                 260
   cgc ctg tac cgt gag gcg cgt gaa tac gtg ggt gat tcg cag gag gtg      1171
   Arg Leu Tyr Arg Glu Ala Arg Glu Tyr Val Gly Asp Ser Gln Glu Val
               265                 270                 275
   gag gct gac cgc ccg gtg gac agg gtg tgg gct gaa acc tcc gag cca      1219
   Glu Ala Asp Arg Pro Val Asp Arg Val Trp Ala Glu Thr Ser Glu Pro
           280                 285                 290
   cgc ctg tct cgc acg gcc atg gcg cgg gtt gac ccc gac acg cag tgg      1267
   Arg Leu Ser Arg Thr Ala Met Ala Arg Val Asp Pro Asp Thr Gln Trp
       295                 300                 305
   aag cca gac ctg tac ctg ggg ttg ccg tac ggc ccc aac gcc gcc caa      1315
   Lys Pro Asp Leu Tyr Leu Gly Leu Pro Tyr Gly Pro Asn Ala Ala Gln
   310                 315                 320                 325
   atc gtg gcc cag ccg tta gat gtg cgg gag gcg agc cac ttt agc aac      1363
   Ile Val Ala Gln Pro Leu Asp Val Arg Glu Ala Ser His Phe Ser Asn
                   330                 335                 340
   cta gcc ccg tgg atc ata ggt gtg ctc aac ggg aca acg ggg gcg ttc      1411
   Leu Ala Pro Trp Ile Ile Gly Val Leu Asn Gly Thr Thr Gly Ala Phe
               345                 350                 355
   aag ggg gat gca ctg atg ctt tcg gcc aaa acc acc ccc cac gtt gac      1459
   Lys Gly Asp Ala Leu Met Leu Ser Ala Lys Thr Thr Pro His Val Asp
           360                 365                 370
   gat ggg tgg ctc ggc aca caa gct ttg acc aga cac gac ata gac gtc      1507
   Asp Gly Trp Leu Gly Thr Gln Ala Leu Thr Arg His Asp Ile Asp Val
       375                 380                 385
   agg cta gca gtc gcg gat acg gcc tgc aca gtg tcc gtg tgg gtg ggt      1555
   Arg Leu Ala Val Ala Asp Thr Ala Cys Thr Val Ser Val Trp Val Gly
   390                 395                 400                 405
   gtg gat tat gtg gct ggg aaa ccg gta ctg cat cac atg gct gtg gcg      1603
   Val Asp Tyr Val Ala Gly Lys Pro Val Leu His His Met Ala Val Ala
                   410                 415                 420
   ggg tat cgt gcc gcg aag ccg cca gtg cgg cgc ctg gca cta gct ctg      1651
   Gly Tyr Arg Ala Ala Lys Pro Pro Val Arg Arg Leu Ala Leu Ala Leu
               425                 430                 435
   tgc gag gcg ctg aca tac cat gtt gcg gtg ggt ggg gtt ctg cca gtg      1699
   Cys Glu Ala Leu Thr Tyr His Val Ala Val Gly Gly Val Leu Pro Val
           440                 445                 450
   aat gcg gtg cat aag cga cag tgc tcc aac tac act gat ata atg tcc      1747
   Asn Ala Val His Lys Arg Gln Cys Ser Asn Tyr Thr Asp Ile Met Ser
       455                 460                 465
   gct gaa gcg tat agt gac ccg ccg gcg cct aac caa ctg ggc ccg cgt      1795
   Ala Glu Ala Tyr Ser Asp Pro Pro Ala Pro Asn Gln Leu Gly Pro Arg
   470                 475                 480                 485
   gtg gct act aac cct ccg gag ggc tgc ctg tcc cag ctg gtg cag gtg      1843
   Val Ala Thr Asn Pro Pro Glu Gly Cys Leu Ser Gln Leu Val Gln Val
                   490                 495                 500
   cac cag tac gac gtg cag act ggg gtt gca aca ggg gtc atg gca gcg      1891
   His Gln Tyr Asp Val Gln Thr Gly Val Ala Thr Gly Val Met Ala Ala
               505                 510                 515
   aga gac gtg cca cct gcc gcg ttc tac ttc ggc ggg acg ccc aac ggc      1939
   Arg Asp Val Pro Pro Ala Ala Phe Tyr Phe Gly Gly Thr Pro Asn Gly
           520                 525                 530
   cgg aac aca ggg tac gct atc gcg cga act gaa gta gat ggc gtg atg      1987
   Arg Asn Thr Gly Tyr Ala Ile Ala Arg Thr Glu Val Asp Gly Val Met
       535                 540                 545
   act cac gtg ctc acg ggc gca gta cac atg aag gga cac ccc gcg ttc      2035
   Thr His Val Leu Thr Gly Ala Val His Met Lys Gly His Pro Ala Phe
   550                 555                 560                 565
   ctg ctc tca ggg cgt aga cgc cag ccg tgc gtc agg gac ggg ttg tac      2083
   Leu Leu Ser Gly Arg Arg Arg Gln Pro Cys Val Arg Asp Gly Leu Tyr
                   570                 575                 580
   tcg ccg ggc gct gtg tcg aag ctg ttc ggc gac ggg agc ggc ggc ctg      2131
   Ser Pro Gly Ala Val Ser Lys Leu Phe Gly Asp Gly Ser Gly Gly Leu
               585                 590                 595
   tgt atg agg gcg gaa gag gcc tcg gga gtg gac cgc ggc ctt cgc tcc      2179
   Cys Met Arg Ala Glu Glu Ala Ser Gly Val Asp Arg Gly Leu Arg Ser
           600                 605                 610
   gtg tgg tcc gac gcc ttg tca aac gct aac aaa act gcc gct gtc cgc      2227
   Val Trp Ser Asp Ala Leu Ser Asn Ala Asn Lys Thr Ala Ala Val Arg
       615                 620                 625
   tca cgc gac ccg ctg tcg gct gcg aac ctc ctg atg gcc ata gca aag      2275
   Ser Arg Asp Pro Leu Ser Ala Ala Asn Leu Leu Met Ala Ile Ala Lys
   630                 635                 640                 645
   ctt aac gga tgg caa gcg aaa ccg gcc ggg ccg cac agc gtc acc gcc      2323
   Leu Asn Gly Trp Gln Ala Lys Pro Ala Gly Pro His Ser Val Thr Ala
                   650                 655                 660
   gac act gac caa ggc caa gtg cgg gtg atc gtt gag ttt tgg cca acg      2371
   Asp Thr Asp Gln Gly Gln Val Arg Val Ile Val Glu Phe Trp Pro Thr
               665                 670                 675
   tct ggt cca cgt tgg ttg gag gtg ctg tca ttc gac gag gag gta gat      2419
   Ser Gly Pro Arg Trp Leu Glu Val Leu Ser Phe Asp Glu Glu Val Asp
           680                 685                 690
   ctt ggg cca gag ggc gac gat ggg caa tgc gat cat cca tcc gag ctg      2467
   Leu Gly Pro Glu Gly Asp Asp Gly Gln Cys Asp His Pro Ser Glu Leu
       695                 700                 705
   gcc ctc gat tat tgg ctg tcg ggt ttg gca agg tac atg ctg aag gac      2515
   Ala Leu Asp Tyr Trp Leu Ser Gly Leu Ala Arg Tyr Met Leu Lys Asp
   710                 715                 720                 725
   ata acc agg aat ggg tac cta ctt aag ggc tgc ggc aag tat gca cgc      2563
   Ile Thr Arg Asn Gly Tyr Leu Leu Lys Gly Cys Gly Lys Tyr Ala Arg
                   730                 735                 740
   aac gag ctg tct ccg ttc caa gcc caa cac aca gcg gcg ccc caa gcg      2611
   Asn Glu Leu Ser Pro Phe Gln Ala Gln His Thr Ala Ala Pro Gln Ala
               745                 750                 755
   gcg tgc gat gtg gtg gtg agc gcg tgg cgg gtg gag acc cgg gtc ggc      2659
   Ala Cys Asp Val Val Val Ser Ala Trp Arg Val Glu Thr Arg Val Gly
           760                 765                 770
   tca gcg aaa gca tct tac atg tac aac ttg ggg gtg gct gtc tgt gca      2707
   Ser Ala Lys Ala Ser Tyr Met Tyr Asn Leu Gly Val Ala Val Cys Ala
       775                 780                 785
   gcc gga gcc gtg gtg gct acg tcg aac ctg gtt gat gcg aac gcg gag      2755
   Ala Gly Ala Val Val Ala Thr Ser Asn Leu Val Asp Ala Asn Ala Glu
   790                 795                 800                 805
   cag cgg cgc agt gca gtg gtg ggc aac ggg aac gtg ctg gcg gcg tat      2803
   Gln Arg Arg Ser Ala Val Val Gly Asn Gly Asn Val Leu Ala Ala Tyr
                   810                 815                 820
   gat agg cgc aca cga ccc gac gcc gca cac gct gac ctg cta gac gtg      2851
   Asp Arg Arg Thr Arg Pro Asp Ala Ala His Ala Asp Leu Leu Asp Val
               825                 830                 835
   ctg aag ggc cta tca aga ttg gcc atg gca ggg tca gtg cgc gtg tac      2899
   Leu Lys Gly Leu Ser Arg Leu Ala Met Ala Gly Ser Val Arg Val Tyr
           840                 845                 850
   agt gca acc cag gcc ggc cac gat cag gca gac gtg aac gtg ctg aga      2947
   Ser Ala Thr Gln Ala Gly His Asp Gln Ala Asp Val Asn Val Leu Arg
       855                 860                 865
   agc gat ggc aag agt ggg ccg agc gca aga att atg gag ata ggg acg      2995
   Ser Asp Gly Lys Ser Gly Pro Ser Ala Arg Ile Met Glu Ile Gly Thr
   870                 875                 880                 885
   tta gta gag cgg cga ggc agc gca gca gat gcc ggg atg gat gcg gca      3043
   Leu Val Glu Arg Arg Gly Ser Ala Ala Asp Ala Gly Met Asp Ala Ala
                       890                 895                 900
   ggg gct gcg gta tcg agc ggc gtg gcg gca gac gac ggc cag ccc gtg      3091
   Gly Ala Ala Val Ser Ser Gly Val Ala Ala Asp Asp Gly Gln Pro Val
                   905                 910                 915
   aga ctg gcg ggg act gat gag ctg tgg tcg acg ttg cgg cgg tta gtg      3139
   Arg Leu Ala Gly Thr Asp Glu Leu Trp Ser Thr Leu Arg Arg Leu Val
               920                 925                 930
   agg tgagcgcagg aggccagctc gcctccccca gctggcaacc gaagatatgc           3192
   Arg
   ggtgataacc gcttaagcgc ggtcgccagc atgaacttct tacgtgtcaa cgtaagtacc    3252
<210> 3
   <211> 2875
   <212> DNA
   <213> Mycovirus MoCV3
<220>
   <221> CDS
   <222> (148)..(2499)
<400> 3
   gcaaaaaaga gaataaagct ttctcctttt tgcaacgatc gtgcgactcc aaactgctgt      60
   gtcgataaca acctttgacg cttgacaagc cgcgctaatt aacaccccac acaacacaac     120
   actccagcca actttactta gtgcaac atg ggc ttg aca ctt gac cca gcg cac     174
                                 Met Gly Leu Thr Leu Asp Pro Ala His
                                 1               5
   cgc tgg cgc tcc acc gac ttg gcg ttc gcg cct gta cgg gag atc agc       222
   Arg Trp Arg Ser Thr Asp Leu Ala Phe Ala Pro Val Arg Glu Ile Ser
   10                  15                  20                  25
   gtg caa acc gcc gtt cgt gga agg gac gac agc gaa gcg gcg ggg aca       270
   Val Gln Thr Ala Val Arg Gly Arg Asp Asp Ser Glu Ala Ala Gly Thr
                   30                  35                  40
   ggg ttc agc ggg gac aag gag act gag gca tgg tac aac ggg tcg gct       318
   Gly Phe Ser Gly Asp Lys Glu Thr Glu Ala Trp Tyr Asn Gly Ser Ala
               45                  50                  55
   cga gtg ccg ata gac gac atg tgt ctt acc gcc ggg atg cgg gca ggt       366
   Arg Val Pro Ile Asp Asp Met Cys Leu Thr Ala Gly Met Arg Ala Gly
           60                  65                  70
   gtc ttg aaa ctg gca gtt gag ata ggc tcc gca aag cca agc tca gcg       414
   Val Leu Lys Leu Ala Val Glu Ile Gly Ser Ala Lys Pro Ser Ser Ala
       75                  80                  85
   gat gag gcg gta gtg cag ctg agg tca gtg cct tac tcg tac gtg ggt       462
   Asp Glu Ala Val Val Gln Leu Arg Ser Val Pro Tyr Ser Tyr Val Gly
   90                  95                  100                 105
   aag cca ctg aca gtg tcg ctt agc cac gcg ggg cgg cat ttc gtg gcg       510
   Lys Pro Leu Thr Val Ser Leu Ser His Ala Gly Arg His Phe Val Ala
                   110                 115                 120
   aga ccg gcg ttg aac gag aca gtg gct atg gcc atg tac gag gcg cct       558
   Arg Pro Ala Leu Asn Glu Thr Val Ala Met Ala Met Tyr Glu Ala Pro
               125                 130                 135
   act gct gac aag tgg gtc gcc gca acc aat ttc aag ctg ccc agg acc       606
   Thr Ala Asp Lys Trp Val Ala Ala Thr Asn Phe Lys Leu Pro Arg Thr
           140                 145                 150
   gtc gcc gcg ccg ggt gct gcg cca caa gtg cct ggc ttg ccg aac ggg       654
   Val Ala Ala Pro Gly Ala Ala Pro Gln Val Pro Gly Leu Pro Asn Gly
       155                 160                 165
   gga gga ggc gcc aat cta ggc ttg ccg aac aat ttt gat gct gtg cga       702
   Gly Gly Gly Ala Asn Leu Gly Leu Pro Asn Asn Phe Asp Ala Val Arg
   170                 175                 180                 185
   cga gtg ctg gta gaa tgc gct cgc ggt gac gat tac ggg tac agg ctg       750
   Arg Val Leu Val Glu Cys Ala Arg Gly Asp Asp Tyr Gly Tyr Arg Leu
                   190                 195                 200
   ttt agc ttg gct aga gtg gtg atg cat gcg gag acg atg cgt agg tca       798
   Phe Ser Leu Ala Arg Val Val Met His Ala Glu Thr Met Arg Arg Ser
               205                 210                 215
   ggc ata tca ccg cgg gag aca ccg gtg atg gcc gat cag aac atg ttc       846
   Gly Ile Ser Pro Arg Glu Thr Pro Val Met Ala Asp Gln Asn Met Phe
           220                 225                 230
   agc ata aca act ggc gac acc ccc cat cta aca gag gcg cag ata aac       894
   Ser Ile Thr Thr Gly Asp Thr Pro His Leu Thr Glu Ala Gln Ile Asn
       235                 240                 245
   aac tac gcc tac gcc tac aac cac act gag caa tcg ccc cag tac cgc       942
   Asn Tyr Ala Tyr Ala Tyr Asn His Thr Glu Gln Ser Pro Gln Tyr Arg
   250                 255                 260                 265
   gcg ttc cta act atg ggc ctt cga ggc gtt ggt cat tat gcc atg ccg       990
   Ala Phe Leu Thr Met Gly Leu Arg Gly Val Gly His Tyr Ala Met Pro
                   270                 275                 280
   ggg aca ata tac tcc gac ggc gac tac ccg gcg gag tgt gcc gcc aac      1038
   Gly Thr Ile Tyr Ser Asp Gly Asp Tyr Pro Ala Glu Cys Ala Ala Asn
               285                 290                 295
   cat cca ata tca ttc gtt cgt gtg ggc ggg ccg cca cca gcg aac gtg      1086
   His Pro Ile Ser Phe Val Arg Val Gly Gly Pro Pro Pro Ala Asn Val
           300                 305                 310
   gct cca gac ccg ccg cac tac acc gct gtg ttg tct aac ccg ggc cta      1134
   Ala Pro Asp Pro Pro His Tyr Thr Ala Val Leu Ser Asn Pro Gly Leu
       315                 320                 325
   gcc ctg tcg tac tac tgg gcg tat gcg tac tca atg ggg cta ggg aga      1182
   Ala Leu Ser Tyr Tyr Trp Ala Tyr Ala Tyr Ser Met Gly Leu Gly Arg
   330                 335                 340                 345
   gtg gcg gga gcg atc cta gcg caa gcg agc ttg gcc ccg cat att tgg      1230
   Val Ala Gly Ala Ile Leu Ala Gln Ala Ser Leu Ala Pro His Ile Trp
                   350                 355                 360
   ggt tcg gct gca gtg gct ccg tac aag aac tgc gcg ccg aag ctg gac      1278
   Gly Ser Ala Ala Val Ala Pro Tyr Lys Asn Cys Ala Pro Lys Leu Asp
               365                 370                 375
   gca gcc gcg tac cta ctg ctg ccg gac cag gag aca gca cac gtc aca      1326
   Ala Ala Ala Tyr Leu Leu Leu Pro Asp Gln Glu Thr Ala His Val Thr
           380                 385                 390
   gct gac agc gcc cgc gag ttg gtc gct aac gcc gct gtg cta tcg gag      1374
   Ala Asp Ser Ala Arg Glu Leu Val Ala Asn Ala Ala Val Leu Ser Glu
       395                 400                 405
   gct tac ctg gct ggc ata ggg gcc aca cta atg agc gcg aga gat ggt      1422
   Ala Tyr Leu Ala Gly Ile Gly Ala Thr Leu Met Ser Ala Arg Asp Gly
   410                 415                 420                 425
   ggg cat caa gac act gct ctg atg atg cgc gcg gtc acg gag aag ttg      1470
   Gly His Gln Asp Thr Ala Leu Met Met Arg Ala Val Thr Glu Lys Leu
                   430                 435                 440
   tct gat ccg gag act agg cgt ggg gcc atg ttg tcg ata acc agc cga      1518
   Ser Asp Pro Glu Thr Arg Arg Gly Ala Met Leu Ser Ile Thr Ser Arg
               445                 450                 455
   ctg tgc cca ggc ggt gtg ggg atg gag tgg ttg agt ccg ttc agc tac      1566
   Leu Cys Pro Gly Gly Val Gly Met Glu Trp Leu Ser Pro Phe Ser Tyr
           460                 465                 470
   gac gtc ttg gat ggc acg gaa cgg tgc ata cgc gcc tgg cgc aac cac      1614
   Asp Val Leu Asp Gly Thr Glu Arg Cys Ile Arg Ala Trp Arg Asn His
       475                 480                 485
   ggg ttt ttg ctg gcg ctg tat gac act tca cct gtg gcc gcg ctg gca      1662
   Gly Phe Leu Leu Ala Leu Tyr Asp Thr Ser Pro Val Ala Ala Leu Ala
   490                 495                 500                 505
   ccg ctg ttt tcc aca ggc gtc gca atg aac aac tca ctt ctg gac cgg      1710
   Pro Leu Phe Ser Thr Gly Val Ala Met Asn Asn Ser Leu Leu Asp Arg
                   510                 515                 520
   aag agc gta gtg acc ggg gct gaa tac ccg cag ttg gtg gcg tgc gcc      1758
   Lys Ser Val Val Thr Gly Ala Glu Tyr Pro Gln Leu Val Ala Cys Ala
               525                 530                 535
   ctc gcg ggc agg gca gag cta gct ggg cgg tgc gag aag ccg tcg cag      1806
   Leu Ala Gly Arg Ala Glu Leu Ala Gly Arg Cys Glu Lys Pro Ser Gln
           540                 545                 550
   gct tac ctg gct gcc cta gca ggc cac agt gcg cgg atg aga gcg tgg      1854
   Ala Tyr Leu Ala Ala Leu Ala Gly His Ser Ala Arg Met Arg Ala Trp
       555                 560                 565
   acg gtg gtg gtg acg gtg ctg gga gtg gtc ccc ccg gca tcg gac gat      1902
   Thr Val Val Val Thr Val Leu Gly Val Val Pro Pro Ala Ser Asp Asp
   570                 575                 580                 585
   gaa gac ctc gct gac gcg tac gag cag gtg gta agc agg caa gaa tca      1950
   Glu Asp Leu Ala Asp Ala Tyr Glu Gln Val Val Ser Arg Gln Glu Ser
                   590                 595                 600
   tcc agt agc gca aga cct cag agt agt cag agt gac agg agt gtt gtg      1998
   Ser Ser Ser Ala Arg Pro Gln Ser Ser Gln Ser Asp Arg Ser Val Val
               605                 610                 615
   cgc ggc cac ggt gca cag gag caa aca ccg tcg ggt gcg gcg ccg tcg      2046
   Arg Gly His Gly Ala Gln Glu Gln Thr Pro Ser Gly Ala Ala Pro Ser
           620                 625                 630
   agc ccg ccg cca atc gcc ccg ctg aga ggc atg cgc ccg ggg tcg cgt      2094
   Ser Pro Pro Pro Ile Ala Pro Leu Arg Gly Met Arg Pro Gly Ser Arg
       635                 640                 645
   gca cgt agc agc aaa ggc tcc tta tct gtg ccg aag ggc caa tta ccg      2142
   Ala Arg Ser Ser Lys Gly Ser Leu Ser Val Pro Lys Gly Gln Leu Pro
   650                 655                 660                 665
   gag gtc ggg gag gag cca gca gag cgg ccg ttg gat gat aga gcg gag      2190
   Glu Val Gly Glu Glu Pro Ala Glu Arg Pro Leu Asp Asp Arg Ala Glu
                   670                 675                 680
   ccc gag ccg cct gct gcc tta agt ccg ccc aag ctg gct ctc gac aag      2238
   Pro Glu Pro Pro Ala Ala Leu Ser Pro Pro Lys Leu Ala Leu Asp Lys
               685                 690                 695
   cca tcg tgg ggt agc tgg gcg tcg gag gtg gca tca gtg gag gct agg      2286
   Pro Ser Trp Gly Ser Trp Ala Ser Glu Val Ala Ser Val Glu Ala Arg
           700                 705                 710
   ctc atc ggt ccg atc agc ggg ccg aaa ggg cag att gtc gaa cct gag      2334
   Leu Ile Gly Pro Ile Ser Gly Pro Lys Gly Gln Ile Val Glu Pro Glu
       715                 720                 725
   tac agg ggg ata gtc cca tcc cgc ggc acg aca acc gcc gcg tcc atg      2382
   Tyr Arg Gly Ile Val Pro Ser Arg Gly Thr Thr Thr Ala Ala Ser Met
   730                 735                 740                 745
   gca tcc ggg act gtg gtg tcg gtg ggc agg cgg gcc aag ggt aag gag      2430
   Ala Ser Gly Thr Val Val Ser Val Gly Arg Arg Ala Lys Gly Lys Glu
                   750                 755                 760
   cca gag aga gcg caa tcg gct agc agt agt tct gaa ccc gcc agc gga      2478
   Pro Glu Arg Ala Gln Ser Ala Ser Ser Ser Ser Glu Pro Ala Ser Gly
               765                 770                 775
   ggg tcg cgc ggg gag gag ttg tagcctgcgc cgacgaggac tagagcggtc         2529
   Gly Ser Arg Gly Glu Glu Leu
           780
   aaggtacgcc tgttcgagag cgggcgagtg gctgcatcac gcaagacgtg aagggcagct    2589
   gggtcgcagg ctggcccgtc cggagtgggc gcttggggag aggcagccca gccggtggtt    2649
   ttggctagtg catcggcgac acgtgtccgc tgaactggtc cagccgtatg gtgaggccac    2709
   atacatgtgc cctgccgccg tgtcgggcac gacggcgttg gacagggcgg tgtgggggtt    2769
   cgagcgcagc cgcaggtgat gcgctcagcc gccaacccca taattagcgc gcccgcaagt    2829
   tagcgcgcac acatcttgtt cgagacgtag ggtcccaaca agtacc                   2875
<210> 4
   <211> 2997
   <212> DNA
   <213> Mycovirus MoCV3
<220>
   <221> CDS
   <222> (163)..(2595)
<400> 4
   gcaaaaaaga gaataaagct ttctcctttt tgcacaacgc acacgatcgt gaaacacttc      60
   aacgttcagc atcgcacgag cagccgtttt gatcatacaa cttcgaactg cgcgaccgac     120
   aggctaggca cgtagttgca gtgtcgtagg taaataacta aa atg gca atg ggc        174
                                                  Met Ala Met Gly
                                                  1
   atg aca ggc ggt cag cta gcg tcg aag ctg cgt atc gac caa ggg ttg       222
   Met Thr Gly Gly Gln Leu Ala Ser Lys Leu Arg Ile Asp Gln Gly Leu
   5                   10                  15                  20
   ggg ttg cct gat gcg ttc ttg aaa cag agt ggg cgg gcc ggc gag gct       270
   Gly Leu Pro Asp Ala Phe Leu Lys Gln Ser Gly Arg Ala Gly Glu Ala
                   25                  30                  35
   gac agt ggc gct gac ggc gag tgg gcc gct ttc ggg ata cac tct ggg       318
   Asp Ser Gly Ala Asp Gly Glu Trp Ala Ala Phe Gly Ile His Ser Gly
               40                  45                  50
   gct ccc ccc aac gcc gcc cac ccc cac gtg gtg cct gcc gca cta gat       366
   Ala Pro Pro Asn Ala Ala His Pro His Val Val Pro Ala Ala Leu Asp
           55                  60                  65
   gcg tca tcg agc gcc ggg cgg gta gac act ttg cgt cca ctg gtg gga       414
   Ala Ser Ser Ser Ala Gly Arg Val Asp Thr Leu Arg Pro Leu Val Gly
       70                  75                  80
   gac gtg gcc tac agc ctg ttt ttg agg tta gga gag act gac tac gac       462
   Asp Val Ala Tyr Ser Leu Phe Leu Arg Leu Gly Glu Thr Asp Tyr Asp
   85                  90                  95                  100
   gtg acc aaa gat gaa gag gcc agc ccg atg gat gtg agc cac tcc gtg       510
   Val Thr Lys Asp Glu Glu Ala Ser Pro Met Asp Val Ser His Ser Val
                   105                 110                 115
   ata tgc tcg tac gct ctt gag atc gat ggg cgc act gcg ctg acg cgc       558
   Ile Cys Ser Tyr Ala Leu Glu Ile Asp Gly Arg Thr Ala Leu Thr Arg
               120                 125                 130
   gcg gat gtc acg tcg cac tgt gct gtg tac cca ccg atg gcc aag agg       606
   Ala Asp Val Thr Ser His Cys Ala Val Tyr Pro Pro Met Ala Lys Arg
           135                 140                 145
   ggg agt gcc acc ccg gtg tcg gtg acg ggc gcg tta acg acg tca cga       654
   Gly Ser Ala Thr Pro Val Ser Val Thr Gly Ala Leu Thr Thr Ser Arg
       150                 155                 160
   cgc acg agc gcg gcc gcc gcc tgc gag ggg ctg gca atg cac gca ggg       702
   Arg Thr Ser Ala Ala Ala Ala Cys Glu Gly Leu Ala Met His Ala Gly
   165                 170                 175                 180
   tcg ggc aat aac gcg gat gtc agc ctg ggg gtg gcc cgc gag ttc atg       750
   Ser Gly Asn Asn Ala Asp Val Ser Leu Gly Val Ala Arg Glu Phe Met
                   185                 190                 195
   tac gac agg gcg cgg cac caa gag aac ggg ctt gag tct gtg ttt gtg       798
   Tyr Asp Arg Ala Arg His Gln Glu Asn Gly Leu Glu Ser Val Phe Val
               200                 205                 210
   cgt atg tgg ctg gtg cat ctc agt gtg ttg tcg cgg cga ccc gtg aca        846
   Arg Met Trp Leu Val His Leu Ser Val Leu Ser Arg Arg Pro Val Thr
           215                 220                 225
   cag gtg gtg gac cca gct atg ctg gct gct cgg ttc gtg aac atc cta        894
   Gln Val Val Asp Pro Ala Met Leu Ala Ala Arg Phe Val Asn Ile Leu
       230                 235                 240
   gct cca gcg gag agc gac gcc gcc agg gca atg cgc ggg gtc agg ata        942
   Ala Pro Ala Glu Ser Asp Ala Ala Arg Ala Met Arg Gly Val Arg Ile
   245                 250                 255                 260
   aac gct cgc ggg ttg agt gac acg gcg ctg gcg ctg ttg gtg ctg gga        990
   Asn Ala Arg Gly Leu Ser Asp Thr Ala Leu Ala Leu Leu Val Leu Gly
                   265                 270                 275
   tgc agc gac acg tca cag gcg gcg ggg ctg cac tac cgg gca cga cgg       1038
   Cys Ser Asp Thr Ser Gln Ala Ala Gly Leu His Tyr Arg Ala Arg Arg
               280                 285                 290
   tac aag ttc acg cgc tcg gca ttg tcc atg tac ggt atg cag ggg cgg       1086
   Tyr Lys Phe Thr Arg Ser Ala Leu Ser Met Tyr Gly Met Gln Gly Arg
           295                 300                 305
   atc cgc ttg gct gtg gcg ttg gac agg gca gag gtg acc ggt ctc gcg       1134
   Ile Arg Leu Ala Val Ala Leu Asp Arg Ala Glu Val Thr Gly Leu Ala
       310                 315                 320
   atc gtg tcc tta gca gag aga tac ggt gcg gag gcc gca tgc ggt gcc       1182
   Ile Val Ser Leu Ala Glu Arg Tyr Gly Ala Glu Ala Ala Cys Gly Ala
   325                 330                 335                 340
   ggc ctg cag act gcg ctg atg atg tac ggc gtg aac gat agc ggg cgg       1230
   Gly Leu Gln Thr Ala Leu Met Met Tyr Gly Val Asn Asp Ser Gly Arg
                   345                 350                 355
   tac gtg cga cta aag tgc ccg gaa ccc gag tta cat gat gat gtg gcc       1278
   Tyr Val Arg Leu Lys Cys Pro Glu Pro Glu Leu His Asp Asp Val Ala
               360                 365                 370
   acg aca gca ggc ata cga gca ctg tcg gtt aga agc tac tcc gat cta       1326
   Thr Thr Ala Gly Ile Arg Ala Leu Ser Val Arg Ser Tyr Ser Asp Leu
           375                 380                 385
   tcg gat aac cgc ctc ctg tca ctg tcc tta ttt gtc ggt cgg gca tgg       1374
   Ser Asp Asn Arg Leu Leu Ser Leu Ser Leu Phe Val Gly Arg Ala Trp
       390                 395                 400
   cgc cag gca gcc ggg cac cta ctg cgg tcc tcc acc atg cag acg acc       1422
   Arg Gln Ala Ala Gly His Leu Leu Arg Ser Ser Thr Met Gln Thr Thr
   405                 410                 415                 420
   act gca gac ata gac gca gtg gtg aac acc ctg ctg ccc agc caa gga       1470
   Thr Ala Asp Ile Asp Ala Val Val Asn Thr Leu Leu Pro Ser Gln Gly
                   425                 430                 435
   gca ttg ata aag gct gtg ggg tgt gcc cat gcc aga gct atg ggg tgg       1518
   Ala Leu Ile Lys Ala Val Gly Cys Ala His Ala Arg Ala Met Gly Trp
               440                 445                 450
   gtg gcg ccg ttg gtt gac acg gtg tcc tac gtg gag cag aac tac cgc       1566
   Val Ala Pro Leu Val Asp Thr Val Ser Tyr Val Glu Gln Asn Tyr Arg
   455 460 465
   cta ttg tgg gag gag cgg ggc gtg gtg cat tgc ctg gct ctc ggc ttg 1614
   Leu Leu Trp Glu Glu Arg Gly Val Val His Cys Leu Ala Leu Gly Leu
   470 475 480
   cgg gtg ccc aac tcc gtg ctg gag gag gcg acg gcg gtg ata gac gtt 1662
   Arg Val Pro Asn Ser Val Leu Glu Glu Ala Thr Ala Val Ile Asp Val
   485 490 495 500
   cct tat ccg ccc gcc ctg tcg ccg tcc gac gac ccg ctg tca gcc aag 1710
   Pro Tyr Pro Pro Ala Leu Ser Pro Ser Asp Asp Pro Leu Ser Ala Lys
   505 510 515
   cca cgc gcg aac agc ctt gcc act ctt ggg ttg gtg gaa tcc ttg ctg 1758
   Pro Arg Ala Asn Ser Leu Ala Thr Leu Gly Leu Val Glu Ser Leu Leu
   520 525 530
   tcg tcg tca ggt gag agc ttg tgc ggg tcc gct cgg gcg aga gga agg 1806
   Ser Ser Ser Gly Glu Ser Leu Cys Gly Ser Ala Arg Ala Arg Gly Arg
   535 540 545
   cgc cag gcc ggg ctg gtg gct gtg ccc gcg cag gta gtg gct cta gcc 1854
   Arg Gln Ala Gly Leu Val Ala Val Pro Ala Gln Val Val Ala Leu Ala
   550 555 560
   ggg cac cgg gtg cag ttc act cta cta agt gtc gct agt ggc gtg gct 1902
   Gly His Arg Val Gln Phe Thr Leu Leu Ser Val Ala Ser Gly Val Ala
   565 570 575 580
   gtg aaa gtg gag gag ctg ccg aca cgg cca ttg ctg gca gag ccg ctg 1950
   Val Lys Val Glu Glu Leu Pro Thr Arg Pro Leu Leu Ala Glu Pro Leu
   585 590 595
   cag acc gga ctg cag gct gtt gag tac gtg cag gcg cct tgg gcc ccg 1998
   Gln Thr Gly Leu Gln Ala Val Glu Tyr Val Gln Ala Pro Trp Ala Pro
   600 605 610
   gcg cag gat gca ccg gaa ccg gcg ccg cct gtg gaa ttt cga gat acg 2046
   Ala Gln Asp Ala Pro Glu Pro Ala Pro Pro Val Glu Phe Arg Asp Thr
   615 620 625
   ctc gat caa ttc cta cat gca tac agg cca agc agg tcc gtg ctg gaa 2094
   Leu Asp Gln Phe Leu His Ala Tyr Arg Pro Ser Arg Ser Val Leu Glu
   630 635 640
   caa cct agg gta gag gtc aca ccc cga atg tcg tca ggg tcg gac gtg 2142
   Gln Pro Arg Val Glu Val Thr Pro Arg Met Ser Ser Gly Ser Asp Val
   645 650 655 660
   ctt aac gtg atg cta ccc gag ggc aca cct gcc tcc ctg cga gag tct 2190
   Leu Asn Val Met Leu Pro Glu Gly Thr Pro Ala Ser Leu Arg Glu Ser
   665 670 675
   ata ata ggc ggg aca tta act cca gtc agt ggc gca ggc gtt ggc ggc 2238
   Ile Ile Gly Gly Thr Leu Thr Pro Val Ser Gly Ala Gly Val Gly Gly
   680 685 690
   tgt gcg gag gca ata gcg aac tcc ctg caa gcg cag cac ggg gtg aca 2286
   Cys Ala Glu Ala Ile Ala Asn Ser Leu Gln Ala Gln His Gly Val Thr
   695 700 705
   gta tct gct ggt gag ata gag aag gcg atg cag ggc gac agc ggc ggt 2334
   Val Ser Ala Gly Glu Ile Glu Lys Ala Met Gln Gly Asp Ser Gly Gly
       710                 715                 720
   acc tcg ctg cag gta gaa gca atg gct ggc gct ctg gcc aag ttt ggg       2382
   Thr Ser Leu Gln Val Glu Ala Met Ala Gly Ala Leu Ala Lys Phe Gly
   725                 730                 735                 740
   gac tac cga ctg gtg tta cta gat gaa cac tct cag ggg gtg agt ttg       2430
   Asp Tyr Arg Leu Val Leu Leu Asp Glu His Ser Gln Gly Val Ser Leu
                   745                 750                 755
   aag gcg gga gcc agc ggg agc aag cca gta aca ata cat cgc aac ggg       2478
   Lys Ala Gly Ala Ser Gly Ser Lys Pro Val Thr Ile His Arg Asn Gly
               760                 765                 770
   tca tcc tat aat gcg ctg ggg cgc ggc ccg ggg cga ggg ata cgc gtc       2526
   Ser Ser Tyr Asn Ala Leu Gly Arg Gly Pro Gly Arg Gly Ile Arg Val
           775                 780                 785
   agg ctg cgt ggc ggt ggc ccg cct gcg cgt gaa gca cag ccg cag cta       2574
   Arg Leu Arg Gly Gly Gly Pro Pro Ala Arg Glu Ala Gln Pro Gln Leu
       790                 795                 800
   agg cgc cta tcg cgc agc agc tgagctgcca cttggccgtg tgcgcgtagc          2625
   Arg Arg Leu Ser Arg Ser Ser
   805                 810
   cgtggctagc ggcggtgccg gagacgtgag tcggctgatt cggtgggcgg tgggcgctga    2685
   tcgtcgtgat tgctagcacg cggggctagt gaggtagtga cgcaccccgt attaggtata    2745
   ggccgctgac gggtggtgca ctgaccgagt gggtgacggt tggggccgcc gccggtaggg    2805
   ataggtccac gccgcggtgg cacacctctt gttgaacatg cagcttcgac aggtaccaca    2865
   acgcaccccc tgcagcgcac gggactccaa cgagcaccac caggtaggtg ccggcggggg    2925
   gcgtcgccgg cgcgcgcagg catgcgacgc ggtgatacac ttcttgttaa acctagggtt    2985
   ctaacaagta cc                                                        2997
<210> 5
   <211> 2861
   <212> DNA
   <213> Mycovirus MoCV3
<220>
   <221> CDS
   <222> (162)..(2159)
<400> 5
   gcattctcct ttttgcaaca caccaagtga gagccacgcg accgccttac gctagaccga      60
   ccgcgctaaa acagctgtgg aggtatccac tacgtcaagc aattagacgg cgttagactg     120
   gaacagttga taaccggctc gtcttacttc tagcaacagc g atg gac gcc aag cag     176
                                                 Met Asp Ala Lys Gln
                                                 1               5
   tcg ggc gcc ggc gcg cgc gtg tcg gaa tgg tgg cca gac cgg caa gtg       224
   Ser Gly Ala Gly Ala Arg Val Ser Glu Trp Trp Pro Asp Arg Gln Val
                   10                  15                  20
   atc agc cga gag gcg ttg cgt caa cgc acg tcc cgg acg ctc ggg gcg         272
   Ile Ser Arg Glu Ala Leu Arg Gln Arg Thr Ser Arg Thr Leu Gly Ala
               25                  30                  35
   atg ggc ggc gcg gtg ggg gtg agt gac acg ccg cgc agc ttc ttc ggt         320
   Met Gly Gly Ala Val Gly Val Ser Asp Thr Pro Arg Ser Phe Phe Gly
           40                  45                  50
   ggg ggc gag gtg ccc act gag cgg ctg agg cgc gga ttg cca agc gaa         368
   Gly Gly Glu Val Pro Thr Glu Arg Leu Arg Arg Gly Leu Pro Ser Glu
       55                  60                  65
   gtg gcc ggt atg ttc act gct gag caa ctc agc gaa atg cta gac atg         416
   Val Ala Gly Met Phe Thr Ala Glu Gln Leu Ser Glu Met Leu Asp Met
   70                  75                  80                  85
   tca gcg gag cag cgc gtt aag gcg ctg gcc gac gca tgg gag cgt ctg         464
   Ser Ala Glu Gln Arg Val Lys Ala Leu Ala Asp Ala Trp Glu Arg Leu
                   90                  95                  100
   gcc aca gcc gaa gcc agc aca ggg ccc acg tca agt gga ctg cag gcg         512
   Ala Thr Ala Glu Ala Ser Thr Gly Pro Thr Ser Ser Gly Leu Gln Ala
               105                 110                 115
   ccg gca cgc cgg gcc ccc agg agc gtc agc gac tac aac acc aca caa         560
   Pro Ala Arg Arg Ala Pro Arg Ser Val Ser Asp Tyr Asn Thr Thr Gln
           120                 125                 130
   tgg gtt gcc gcg gcg gct agc gac ggc ata cga gtg acg ccc aca ctg         608
   Trp Val Ala Ala Ala Ala Ser Asp Gly Ile Arg Val Thr Pro Thr Leu
       135                 140                 145
   acc gcg gct gcg cgc gcg gtg gcg ccc gcc ttg aac agg gca gtg gtg         656
   Thr Ala Ala Ala Arg Ala Val Ala Pro Ala Leu Asn Arg Ala Val Val
   150                 155                 160                 165
   ccg ggc ggg tgc caa ccc cat gcg agt tat ggg cgc gcg aca gtc gct         704
   Pro Gly Gly Cys Gln Pro His Ala Ser Tyr Gly Arg Ala Thr Val Ala
                   170                 175                 180
   acc ggg act ggt gac gta gct ccg ccg tcc gct cgt caa acc gcg gtg         752
   Thr Gly Thr Gly Asp Val Ala Pro Pro Ser Ala Arg Gln Thr Ala Val
               185                 190                 195
   gtg cag gcc att ggt gag agc gtg cct acg gaa ccg gtg gat gcc gag         800
   Val Gln Ala Ile Gly Glu Ser Val Pro Thr Glu Pro Val Asp Ala Glu
           200                 205                 210
   gtg aaa gag ggg acg cct agc ccc gcg gca cca ccc cgc cta ctg tgc         848
   Val Lys Glu Gly Thr Pro Ser Pro Ala Ala Pro Pro Arg Leu Leu Cys
       215                 220                 225
   gga acc gcg act cta ctc gca ctg ccg cag gtt tgc tgt ggg agt gct         896
   Gly Thr Ala Thr Leu Leu Ala Leu Pro Gln Val Cys Cys Gly Ser Ala
   230                 235                 240                 245
   ctg gac cac ggg tgc gcc gcg gcc ccc tgc gcg gtc ggc cag gtg tct         944
   Leu Asp His Gly Cys Ala Ala Ala Pro Cys Ala Val Gly Gln Val Ser
                   250                 255                 260
   ggg gct gcg gcg gcg gtt gcg ttg gcc tcg cgc ccg gtg gtg gag ccc         992
   Gly Ala Ala Ala Ala Val Ala Leu Ala Ser Arg Pro Val Val Glu Pro
               265                 270                 275
   gac gcc gat gcg agc att atg ggc gag cgt tgg aag gtg atg tca aca      1040
   Asp Ala Asp Ala Ser Ile Met Gly Glu Arg Trp Lys Val Met Ser Thr
           280                 285                 290
   ttg gcg gca cga ttg ggc ccg ata gca cag tgc cac ttc ggg tca tgc      1088
   Leu Ala Ala Arg Leu Gly Pro Ile Ala Gln Cys His Phe Gly Ser Cys
       295                 300                 305
   cgc ggt gtg tgt gca tgc acc cac agg cac ccg cta gcc cga gca ctg      1136
   Arg Gly Val Cys Ala Cys Thr His Arg His Pro Leu Ala Arg Ala Leu
   310                 315                 320                 325
   atg ggg atc ggg gta ggc acc gag gaa ctg cgt ggg gcg cgc gta cat      1184
   Met Gly Ile Gly Val Gly Thr Glu Glu Leu Arg Gly Ala Arg Val His
                   330                 335                 340
   cta acg acc ccg caa gcg tca cgg tcc gtg gcc tcc gtc ctg ctt gag      1232
   Leu Thr Thr Pro Gln Ala Ser Arg Ser Val Ala Ser Val Leu Leu Glu
               345                 350                 355
   aag gtc ggc ggg ggc agc cgt gtg gag cgt ctc gcg gtt gag ttc gta      1280
   Lys Val Gly Gly Gly Ser Arg Val Glu Arg Leu Ala Val Glu Phe Val
           360                 365                 370
   aag gct gtg gac ggg cca gtg ctg acc agc ctg gtg gtg gca agc cgc      1328
   Lys Ala Val Asp Gly Pro Val Leu Thr Ser Leu Val Val Ala Ser Arg
       375                 380                 385
   gat ggc aag gcg gac agc ctg ttg acc ttg gtt gct gag gcg atg gca      1376
   Asp Gly Lys Ala Asp Ser Leu Leu Thr Leu Val Ala Glu Ala Met Ala
   390                 395                 400                 405
   caa gga tcg ctg gcg gcg cca cgc acc atg gtg aca ttt ggg agg ggc      1424
   Gln Gly Ser Leu Ala Ala Pro Arg Thr Met Val Thr Phe Gly Arg Gly
                   410                 415                 420
   gac ggc ggt ggg cca gac tta gac ccc aat aaa cta tac gac gcg tgc      1472
   Asp Gly Gly Gly Pro Asp Leu Asp Pro Asn Lys Leu Tyr Asp Ala Cys
               425                 430                 435
   gtg tac tca ggc gag cgg ggc cac aag cta gga gta ctc tct cga gcg      1520
   Val Tyr Ser Gly Glu Arg Gly His Lys Leu Gly Val Leu Ser Arg Ala
           440                 445                 450
   gtg tgg ttg gaa agc gat gcg gat gtc cac tgg atg ccg cat tcc aac      1568
   Val Trp Leu Glu Ser Asp Ala Asp Val His Trp Met Pro His Ser Asn
       455                 460                 465
   tcg ctc aag gtt acg ctg cag tgc tgg aat cgg cgt ggg gac tgc aag      1616
   Ser Leu Lys Val Thr Leu Gln Cys Trp Asn Arg Arg Gly Asp Cys Lys
   470                 475                 480                 485
   ggg gtc agc gcg gtg ata gga cct acg acg tgg cgc ccg cat gtg ggc      1664
   Gly Val Ser Ala Val Ile Gly Pro Thr Thr Trp Arg Pro His Val Gly
                   490                 495                 500
   aat tct tcc tgg gag acg ttg ggg tcg gat gtc tac cgg gac tgg ttc      1712
   Asn Ser Ser Trp Glu Thr Leu Gly Ser Asp Val Tyr Arg Asp Trp Phe
               505                 510                 515
   ggt tta gtc gtc gcg cgc agg ggc ggc ggc gac aca act gtg cgg cac      1760
   Gly Leu Val Val Ala Arg Arg Gly Gly Gly Asp Thr Thr Val Arg His
           520                 525                 530
   caa ttg agc tac aac cca ggc atg gag gcc gac cag ctt ggc ggt ctg      1808
   Gln Leu Ser Tyr Asn Pro Gly Met Glu Ala Asp Gln Leu Gly Gly Leu
       535                 540                 545
   ctg gcg cac tcc ggc tgc ggc gtg tac gtg agt ggc gca gac gat gcg      1856
   Leu Ala His Ser Gly Cys Gly Val Tyr Val Ser Gly Ala Asp Asp Ala
   550                 555                 560                 565
   gca acc gtg gct gca gtg cac ccg tca ctg cag gtt acg tac act cat      1904
   Ala Thr Val Ala Ala Val His Pro Ser Leu Gln Val Thr Tyr Thr His
                   570                 575                 580
   ata aca ctg ggg agt cca acc gct agc aag tta gtg cag gcg gcc acc      1952
   Ile Thr Leu Gly Ser Pro Thr Ala Ser Lys Leu Val Gln Ala Ala Thr
               585                 590                 595
   gca ctg gac gag ttt gtg cgg cga ggg ggg cgt atc gcc cat gag gct      2000
   Ala Leu Asp Glu Phe Val Arg Arg Gly Gly Arg Ile Ala His Glu Ala
           600                 605                 610
   gcg ggg aca gct gac gaa gct gtc ata acc gct agg gac gcg ggc ggt      2048
   Ala Gly Thr Ala Asp Glu Ala Val Ile Thr Ala Arg Asp Ala Gly Gly
       615                 620                 625
   cga cga cag ggg cgc tgt acc aag gcg aag gcc act gcg cgg tca ctc      2096
   Arg Arg Gln Gly Arg Cys Thr Lys Ala Lys Ala Thr Ala Arg Ser Leu
   630                 635                 640                 645
   ggc ccg ata gac gat ggg gcg agc cag gac caa gcg ccg aca cgc ccg      2144
   Gly Pro Ile Asp Asp Gly Ala Ser Gln Asp Gln Ala Pro Thr Arg Pro
                   650                 655                 660
   cgt ggc ggg gcg ttg taggcgtctg ccgctgagaa cagtgcagcg ggggcgctac      2199
   Arg Gly Gly Ala Leu
               665
   gaccaagttg gtggactggc gtactgtggc catggcgtgg gggccacatc aacggcgcgc    2259
   agagcgggtc gtcgcgcgaa ggcgatccgg ggcatacagg cgtgcggaga ccgggcgcga    2319
   tgactggccg gcggtttgcc cgccgtttcg ccggcggcgc agactactgg ggcaagctgc    2379
   tgtagacatc ttggagcggc agggcgcgtc agcagtcgcc accgagttac gcggcgccgg    2439
   aggacccacc ggacgcgtga gcgcgcgtgg gcggctgcgg ggcatgcaag cagcgtgaac    2499
   caccataccg gcgcaggcgg gcacggggat acagccgggg tcaggctcaa taaccgatgg    2559
   gcgatcgccc cgatgcctca caaacggtgc gcgcggcggg ggcaggttgc tgtgtgtact    2619
   tctcaagtaa ccagtgtgtt tgaacatcgc tagcgcggtt aggcccgcgc cccggcggac    2679
   cacctcttgt ttagcataga gcttgaacga gtgtcgcctg gtgagccgtg gtgaacgtcg    2739
   cggggattgg tgaggtggat gcggggcgct aggtcgtctc cccctggtgg cccaaggttt    2799
   gcgacgacgg caaccaacgc gtggtcgtcg gtatgaactt cttacgtgat gacgtaagta    2859
   cc                                                                   2861
<210> 6
   <211> 1127
   <212> PRT
   <213> Mycovirus MoCV3
<400> 6
   Met Ala Gly Gly Tyr Asp Glu Asp Val Gly Ser Leu Gly Ser Gly Glu
   1               5                   10                  15
   Pro Val Phe Gly Ala Ser Tyr Thr Asn Ile Trp His Ser Thr Leu Leu
               20                  25                  30
   Ala Ala Glu Gly Ser Val Ala His Ser Gly Pro Ala Leu Tyr Ala Ile
           35                  40                  45
   Val Leu Pro Leu Ser Cys Gly Lys Ser Ser Leu Ala Ser Val Leu Ser
       50                  55                  60
   Gly Tyr Asp Ile Asp Asp Met Val Val Asn Ser Ala Ala Leu His Ala
   65                  70                  75                  80
   Asp Asp Glu Trp Arg Thr Met Leu Asp Ala Arg Ser Lys Gly Trp Ala
                   85                  90                  95
   Tyr Glu Asp Lys Ala Ala Tyr Arg Leu Ala Asn Asp Leu Met Leu Arg
               100                 105                 110
   Arg Ala Arg Arg Phe Leu Arg Ala Phe Glu Gly Asp Asp Asn Ala Pro
           115                 120                 125
   Val Val Tyr Val His Thr Arg Glu Leu Ala Thr Ala Leu Gly Leu Arg
       130                 135                 140
   Ile Ile Phe Asp Gly Tyr Val Glu Glu Ala Ala Trp Leu Gly Cys Arg
   145                 150                 155                 160
   Arg Gln Leu Glu Ser Asp Ala Val Thr Arg Asp Arg Asp Leu Arg Ala
                   165                 170                 175
   Tyr Arg Gly Gln Val Ala Ala Asn Arg Ala His Ala Ile Arg His Arg
               180                 185                 190
   Gln Pro Glu Pro Val Pro Tyr Thr Ser His Ser Arg Leu Ala Glu Ala
           195                 200                 205
   Ala Glu Ala Ala Ile Thr Arg Ala Gly Leu Cys Ala Gly Ser Pro Arg
       210                 215                 220
   Asp Leu Ala Asp Arg Thr Lys Leu Cys Gly Ala Pro Pro Gln Ile Met
   225 230 235 240
   Leu Asp Leu Ala His Ser Ile Cys Arg Asp Arg His Arg Pro Ala Trp
   245 250 255
   Leu Arg Ala Val Ala Ala Lys Leu Leu Arg Tyr Arg Met Gly Glu Val
   260 265 270
   Leu Pro Gln Glu Ala Leu Ala Ala Asp Asn Tyr Ser Glu Trp Ala Arg
   275 280 285
   Val Ile His Ala Thr Asp Gln His Arg Val Ala Glu Ala Pro Ala Gln
   290 295 300
   Ser Leu Arg Gly Gln Asn Trp Ser Glu Val Phe Pro Tyr Gly Ala Gly
   305 310 315 320
   Asn Ser Arg Phe Ala Leu Val Lys Ile Gly Asp Trp Ile Asp Cys Thr
   325 330 335
   Gly Thr Ser Ala Met Gly Phe Gly Tyr Glu Trp Phe Arg Gln Met Val
   340 345 350
   Thr Arg Arg Glu Gly Thr Tyr Glu Gln Ala Ser Cys Met Leu Leu Met
   355 360 365
   Gly Asp Val Phe Asp Tyr Met Ala Pro Glu Leu His Pro Leu Ile Gln
   370 375 380
   Arg Leu Pro Met Gly Ser Leu Arg Leu Glu His Tyr Ala Glu Ile Ala
   385 390 395 400
   Lys Glu Ile His Arg Leu Val Arg Ser Ser Val Thr Leu Leu Gly Arg
   405 410 415
   Arg Leu Asp Ala Gly Gln Leu Ser Val Cys Thr Tyr Trp Asp Cys Leu
   420 425 430
   Ala Gly Arg Tyr Leu Gly Ser Gly Asp Met Glu Lys Glu Leu Ala Asp
   435 440 445
   Arg Thr Ser Glu Gln Lys Pro Arg Val Trp Val Ser Arg Asp Gly Thr
   450 455 460
   Gln Ser Ala Asp Arg Phe Ala His Glu Phe Ala Cys Glu Val Arg Ala
   465 470 475 480
   Leu Leu His Gln Thr Ile Ala Asp Gly Gly Glu Gln Met Arg Thr Val
   485 490 495
   Thr Asp Met Val Ala Ser Phe Asp Thr Phe Leu Glu Tyr Arg Lys Lys
   500 505 510
   Trp Val Arg Pro Gly Ser Val Thr Gly Ser Pro Lys Ala Asp Ile Tyr
   515 520 525
   Leu Glu Ala Val Ser Glu Arg Glu Gly Met Ile Ala Glu Val Ala Asp
   530 535 540
   Asp Ile Ala Ala Met Gly Thr Tyr Val Leu Ala Asn Val Arg Leu Asn
   545 550 555 560
   Lys Ala Ala Thr Phe Glu Phe Ala Glu Phe Pro Ala Ile Val Lys Arg
   565 570 575
   Val Leu Ala Asp Tyr Val Pro Asn Ser Phe Thr Arg Tyr Phe Ile Lys
   580 585 590
   Asn Glu Ile Gly Lys Pro Ala Gly Arg Ala Leu Tyr Pro Ser His Leu
   595 600 605
   Ala His Tyr Val Ala Gly Gln Phe Ala Leu Tyr Ala Leu Met Lys Ala
   610 615 620
   Gln Pro Ile Pro Lys Val Arg Leu Ala Ser Glu Arg Asp Val Ala Met
   625 630 635 640
   Asp Glu His Trp Met Trp Met Gln Ala Arg Glu Phe Thr Val Gly Val
   645 650 655
   Met Leu Asp Tyr Asp Asn Phe Asn Glu Lys His Glu Phe Ala Asp Met
   660 665 670
   Gln Leu Ile Met Arg Glu Leu Lys Gly Leu Tyr Arg Thr Ala Gly Val
   675 680 685
   Leu Ser Pro Asp Leu Lys Thr Met Ile Asp Trp Val Ala Glu Ala Tyr
   690 695 700
   Asp Arg Thr Val Leu Glu Tyr Asp Gly Glu Leu His Ser Phe Lys His
   705 710 715 720
   Gly Met Leu Ser Gly Gln Ala Pro Thr Ser Ala Ile Asn Asn Ile Ile
   725 730 735
   Asn Gly Ala Asn Lys Arg Leu Leu Ile Arg Gln Val Glu Glu Leu Thr
   740 745 750
   Gly Arg Val Ile Phe Gln Lys Arg Thr Ser Gly Gly Asp Asp Val Ala
   755 760 765
   Gly Glu Thr Tyr Ser Leu Tyr Asp Ala Tyr Leu Ala Val Lys Cys Gly
   770 775 780
   Gln Gln Met Gly Leu Ala Phe Lys Asp Ile Lys Gln Leu Leu Ser Ser
   785 790 795 800
   Asp Tyr Tyr Glu Phe Phe Arg Leu Phe Val Ser Val Lys Gly Val His
   805 810 815
   Gly Ser Leu Pro Arg Ala Leu Gly Ser Ile Cys Ser Gly Gln Trp Ser
   820 825 830
   Asn Ser Val Lys Ala Lys Phe Val Asp Pro Ala Ala Lys Leu Ser Ser
   835 840 845
   Val Thr Asp Ala Ala Phe Lys Ile Ala Arg Arg Ala Gly Gly Asn Ala
   850 855 860
   Thr Phe Arg Glu Lys Leu Cys Ala Thr Ala Phe Lys Lys Trp Ala Ser
   865 870 875 880
   Tyr Asn Glu Gln Ala Leu Val Arg Gly Phe Ile His Gly Glu Arg His
   885 890 895
   Ser Gly Gly Leu Gly Val Pro Met Ser Asp Gly Ser Val Leu Arg Ile
   900 905 910
   Glu Pro Ile Gln Trp Pro Asp Glu Glu Arg Val Arg Leu Lys Gly Leu
   915 920 925
   Pro Lys Asp Ala Ser Gln Val Val Val Glu Asp Ala Val Lys Gln Ala
   930 935 940
   Thr Glu Leu Val Gly Pro Asp Ser Val Glu Ser Ala Glu Val Val Ala
   945 950 955 960
   Asn Arg Leu Ser Glu Gln Val Phe Lys Ala Asn Val Ala Ala Met Glu
   965 970 975
   Gly Ser Arg Val Gly Gln Leu Leu Gly Ser Trp Glu Gly Pro Arg His
               980                 985                 990
   Val Arg Val His Glu Val Leu Arg  Ile Ser Glu Ala Asp  Val Ala Ala
           995                 1000                 1005
   Thr Ala  Pro Thr Ala Glu Glu  Phe Arg Ala Ala Tyr  Ala Lys His
       1010                 1015                 1020
   Lys Thr  Ile Ile Glu Tyr Tyr  Arg Lys Ala Gly Ala  Lys Tyr Asp
       1025                 1030                 1035
   Ala Leu  Ala Gly Val Val Lys  Pro Lys Ala Arg Glu  Lys Leu Ala
       1040                 1045                 1050
   Arg Ala  Ser Cys Asn Gly Thr  Pro Cys Asp Tyr Lys  Lys Leu Tyr
       1055                 1060                 1065
   Phe Trp  Lys Glu His Leu Thr  Met Tyr Gly Cys Gly  Thr Tyr Leu
       1070                 1075                 1080
   Leu Thr  Glu Asp Thr Tyr Asp  Ala Ala Ser Met Leu  Ala Leu Val
       1085                 1090                 1095
   Val Ser  Ser Glu Leu Ser Asn  Glu Ala Val Ser Arg  Arg Leu Ala
       1100                 1105                 1110
   Glu Cys  Ala Val Ala Leu Lys  Arg Ala Gly Leu Val  Ser Tyr
       1115                 1120                 1125
<210> 7
   <211> 934
   <212> PRT
   <213> Mycovirus MoCV3
<400> 7
   Met Leu Met Arg Glu Ser Arg Gly Met Glu Glu Glu Val Ala Ser Ser
   1               5                   10                  15
   Arg Glu Ala Leu Gln Gly Trp Gly Ala Ser Pro Ala Ala Gly Trp Pro
               20                  25                  30
   Asn Arg Glu Thr Asn Gly Tyr Ala Gln Asp Arg Arg Pro Gly Ala Gln
           35                  40                  45
   Ala Pro Leu Thr Gln Glu Ser Ala Trp Asp Leu Gly Ser Trp Thr Ser
       50                  55                  60
   His Thr Glu His Gln Pro Ser Met Ser Arg Asn Ala Met Leu Gly Ser
   65 70 75 80
   Ala Val Gly Ala Gly Arg Ser Thr Gly Thr Asn Glu Ala Arg Arg Ser
   85 90 95
   Thr Ala Glu Pro Ala Ala Gly Ala Arg Ala His Gly Pro Ser Gln Pro
   100 105 110
   Gly Asn Leu Leu Pro Ser Thr Ser Val Tyr Arg Ser Gly Arg Met Glu
   115 120 125
   Gln Pro Thr Gln Ser Arg Arg Tyr Pro Ala Pro Leu Arg Thr Lys Pro
   130 135 140
   Thr Lys Ser Gln Pro His Val Glu Gln Ala Gly Gly Tyr Thr Arg Gln
   145 150 155 160
   Arg Ala Thr Ala Thr Asp Val Leu Ser Asp Ala Gly Gly Gly His Pro
   165 170 175
   Thr Gln Arg Pro Leu Pro Arg Arg Gly Ala Gly Phe Asp Thr Ala Met
   180 185 190
   Gly His Ser Pro Tyr Glu Pro Ser Ala Glu Leu Pro Thr Glu Ser Gln
   195 200 205
   Gln Ser Tyr Ala Leu Ser Pro Gly Ser Phe Gln Asp Asp Ala Leu Ala
   210 215 220
   Ala Leu Ser Thr Leu Gly Lys Val Pro Ala Leu Glu Val Ala Gly Ile
   225 230 235 240
   Val Arg Arg Gly Ala Thr Ile Leu Gly Lys Leu Glu Pro Pro Ser Glu
   245 250 255
   Glu Gln Thr Tyr Ala Arg Leu Tyr Arg Glu Ala Arg Glu Tyr Val Gly
   260 265 270
   Asp Ser Gln Glu Val Glu Ala Asp Arg Pro Val Asp Arg Val Trp Ala
   275 280 285
   Glu Thr Ser Glu Pro Arg Leu Ser Arg Thr Ala Met Ala Arg Val Asp
   290 295 300
   Pro Asp Thr Gln Trp Lys Pro Asp Leu Tyr Leu Gly Leu Pro Tyr Gly
   305 310 315 320
   Pro Asn Ala Ala Gln Ile Val Ala Gln Pro Leu Asp Val Arg Glu Ala
   325 330 335
   Ser His Phe Ser Asn Leu Ala Pro Trp Ile Ile Gly Val Leu Asn Gly
   340 345 350
   Thr Thr Gly Ala Phe Lys Gly Asp Ala Leu Met Leu Ser Ala Lys Thr
   355 360 365
   Thr Pro His Val Asp Asp Gly Trp Leu Gly Thr Gln Ala Leu Thr Arg
   370 375 380
   His Asp Ile Asp Val Arg Leu Ala Val Ala Asp Thr Ala Cys Thr Val
   385 390 395 400
   Ser Val Trp Val Gly Val Asp Tyr Val Ala Gly Lys Pro Val Leu His
   405 410 415
   His Met Ala Val Ala Gly Tyr Arg Ala Ala Lys Pro Pro Val Arg Arg
   420 425 430
   Leu Ala Leu Ala Leu Cys Glu Ala Leu Thr Tyr His Val Ala Val Gly
   435 440 445
   Gly Val Leu Pro Val Asn Ala Val His Lys Arg Gln Cys Ser Asn Tyr
   450 455 460
   Thr Asp Ile Met Ser Ala Glu Ala Tyr Ser Asp Pro Pro Ala Pro Asn
   465 470 475 480
   Gln Leu Gly Pro Arg Val Ala Thr Asn Pro Pro Glu Gly Cys Leu Ser
   485 490 495
   Gln Leu Val Gln Val His Gln Tyr Asp Val Gln Thr Gly Val Ala Thr
   500 505 510
   Gly Val Met Ala Ala Arg Asp Val Pro Pro Ala Ala Phe Tyr Phe Gly
   515 520 525
   Gly Thr Pro Asn Gly Arg Asn Thr Gly Tyr Ala Ile Ala Arg Thr Glu
   530 535 540
   Val Asp Gly Val Met Thr His Val Leu Thr Gly Ala Val His Met Lys
   545 550 555 560
   Gly His Pro Ala Phe Leu Leu Ser Gly Arg Arg Arg Gln Pro Cys Val
   565 570 575
   Arg Asp Gly Leu Tyr Ser Pro Gly Ala Val Ser Lys Leu Phe Gly Asp
   580 585 590
   Gly Ser Gly Gly Leu Cys Met Arg Ala Glu Glu Ala Ser Gly Val Asp
   595 600 605
   Arg Gly Leu Arg Ser Val Trp Ser Asp Ala Leu Ser Asn Ala Asn Lys
   610 615 620
   Thr Ala Ala Val Arg Ser Arg Asp Pro Leu Ser Ala Ala Asn Leu Leu
   625 630 635 640
   Met Ala Ile Ala Lys Leu Asn Gly Trp Gln Ala Lys Pro Ala Gly Pro
   645 650 655
   His Ser Val Thr Ala Asp Thr Asp Gln Gly Gln Val Arg Val Ile Val
   660 665 670
   Glu Phe Trp Pro Thr Ser Gly Pro Arg Trp Leu Glu Val Leu Ser Phe
   675 680 685
   Asp Glu Glu Val Asp Leu Gly Pro Glu Gly Asp Asp Gly Gln Cys Asp
   690 695 700
   His Pro Ser Glu Leu Ala Leu Asp Tyr Trp Leu Ser Gly Leu Ala Arg
   705 710 715 720
   Tyr Met Leu Lys Asp Ile Thr Arg Asn Gly Tyr Leu Leu Lys Gly Cys
   725 730 735
   Gly Lys Tyr Ala Arg Asn Glu Leu Ser Pro Phe Gln Ala Gln His Thr
   740 745 750
   Ala Ala Pro Gln Ala Ala Cys Asp Val Val Val Ser Ala Trp Arg Val
   755 760 765
   Glu Thr Arg Val Gly Ser Ala Lys Ala Ser Tyr Met Tyr Asn Leu Gly
   770 775 780
   Val Ala Val Cys Ala Ala Gly Ala Val Val Ala Thr Ser Asn Leu Val
   785 790 795 800
   Asp Ala Asn Ala Glu Gln Arg Arg Ser Ala Val Val Gly Asn Gly Asn
   805 810 815
   Val Leu Ala Ala Tyr Asp Arg Arg Thr Arg Pro Asp Ala Ala His Ala
               820                 825                 830
   Asp Leu Leu Asp Val Leu Lys Gly Leu Ser Arg Leu Ala Met Ala Gly
           835                 840                 845
   Ser Val Arg Val Tyr Ser Ala Thr Gln Ala Gly His Asp Gln Ala Asp
       850                 855                 860
   Val Asn Val Leu Arg Ser Asp Gly Lys Ser Gly Pro Ser Ala Arg Ile
   865                 870                 875                 880
   Met Glu Ile Gly Thr Leu Val Glu Arg Arg Gly Ser Ala Ala Asp Ala
                   885                 890                 895
   Gly Met Asp Ala Ala Gly Ala Ala Val Ser Ser Gly Val Ala Ala Asp
               900                 905                 910
   Asp Gly Gln Pro Val Arg Leu Ala Gly Thr Asp Glu Leu Trp Ser Thr
           915                 920                 925
   Leu Arg Arg Leu Val Arg
       930
<210> 8
   <211> 784
   <212> PRT
   <213> Mycovirus MoCV3
<400> 8
   Met Gly Leu Thr Leu Asp Pro Ala His Arg Trp Arg Ser Thr Asp Leu
   1               5                   10                  15
   Ala Phe Ala Pro Val Arg Glu Ile Ser Val Gln Thr Ala Val Arg Gly
               20                  25                  30
   Arg Asp Asp Ser Glu Ala Ala Gly Thr Gly Phe Ser Gly Asp Lys Glu
           35                  40                  45
   Thr Glu Ala Trp Tyr Asn Gly Ser Ala Arg Val Pro Ile Asp Asp Met
       50                  55                  60
   Cys Leu Thr Ala Gly Met Arg Ala Gly Val Leu Lys Leu Ala Val Glu
   65                  70                  75                  80
   Ile Gly Ser Ala Lys Pro Ser Ser Ala Asp Glu Ala Val Val Gln Leu
                   85                  90                  95
   Arg Ser Val Pro Tyr Ser Tyr Val Gly Lys Pro Leu Thr Val Ser Leu
   100 105 110
   Ser His Ala Gly Arg His Phe Val Ala Arg Pro Ala Leu Asn Glu Thr
   115 120 125
   Val Ala Met Ala Met Tyr Glu Ala Pro Thr Ala Asp Lys Trp Val Ala
   130 135 140
   Ala Thr Asn Phe Lys Leu Pro Arg Thr Val Ala Ala Pro Gly Ala Ala
   145 150 155 160
   Pro Gln Val Pro Gly Leu Pro Asn Gly Gly Gly Gly Ala Asn Leu Gly
   165 170 175
   Leu Pro Asn Asn Phe Asp Ala Val Arg Arg Val Leu Val Glu Cys Ala
   180 185 190
   Arg Gly Asp Asp Tyr Gly Tyr Arg Leu Phe Ser Leu Ala Arg Val Val
   195 200 205
   Met His Ala Glu Thr Met Arg Arg Ser Gly Ile Ser Pro Arg Glu Thr
   210 215 220
   Pro Val Met Ala Asp Gln Asn Met Phe Ser Ile Thr Thr Gly Asp Thr
   225 230 235 240
   Pro His Leu Thr Glu Ala Gln Ile Asn Asn Tyr Ala Tyr Ala Tyr Asn
   245 250 255
   His Thr Glu Gln Ser Pro Gln Tyr Arg Ala Phe Leu Thr Met Gly Leu
   260 265 270
   Arg Gly Val Gly His Tyr Ala Met Pro Gly Thr Ile Tyr Ser Asp Gly
   275 280 285
   Asp Tyr Pro Ala Glu Cys Ala Ala Asn His Pro Ile Ser Phe Val Arg
   290 295 300
   Val Gly Gly Pro Pro Pro Ala Asn Val Ala Pro Asp Pro Pro His Tyr
   305 310 315 320
   Thr Ala Val Leu Ser Asn Pro Gly Leu Ala Leu Ser Tyr Tyr Trp Ala
   325 330 335
   Tyr Ala Tyr Ser Met Gly Leu Gly Arg Val Ala Gly Ala Ile Leu Ala
   340 345 350
   Gln Ala Ser Leu Ala Pro His Ile Trp Gly Ser Ala Ala Val Ala Pro
           355                 360                 365
   Tyr Lys Asn Cys Ala Pro Lys Leu Asp Ala Ala Ala Tyr Leu Leu Leu
       370                 375                 380
   Pro Asp Gln Glu Thr Ala His Val Thr Ala Asp Ser Ala Arg Glu Leu
   385                 390                 395                 400
   Val Ala Asn Ala Ala Val Leu Ser Glu Ala Tyr Leu Ala Gly Ile Gly
                   405                 410                 415
   Ala Thr Leu Met Ser Ala Arg Asp Gly Gly His Gln Asp Thr Ala Leu
               420                 425                 430
   Met Met Arg Ala Val Thr Glu Lys Leu Ser Asp Pro Glu Thr Arg Arg
           435                 440                 445
   Gly Ala Met Leu Ser Ile Thr Ser Arg Leu Cys Pro Gly Gly Val Gly
       450                 455                 460
   Met Glu Trp Leu Ser Pro Phe Ser Tyr Asp Val Leu Asp Gly Thr Glu
   465                 470                 475                 480
   Arg Cys Ile Arg Ala Trp Arg Asn His Gly Phe Leu Leu Ala Leu Tyr
                   485                 490                 495
   Asp Thr Ser Pro Val Ala Ala Leu Ala Pro Leu Phe Ser Thr Gly Val
               500                 505                 510
   Ala Met Asn Asn Ser Leu Leu Asp Arg Lys Ser Val Val Thr Gly Ala
           515                 520                 525
   Glu Tyr Pro Gln Leu Val Ala Cys Ala Leu Ala Gly Arg Ala Glu Leu
       530                 535                 540
   Ala Gly Arg Cys Glu Lys Pro Ser Gln Ala Tyr Leu Ala Ala Leu Ala
   545                 550                 555                 560
   Gly His Ser Ala Arg Met Arg Ala Trp Thr Val Val Val Thr Val Leu
                   565                 570                 575
   Gly Val Val Pro Pro Ala Ser Asp Asp Glu Asp Leu Ala Asp Ala Tyr
               580                 585                 590
   Glu Gln Val Val Ser Arg Gln Glu Ser Ser Ser Ser Ala Arg Pro Gln
           595                 600                 605
   Ser Ser Gln Ser Asp Arg Ser Val Val Arg Gly His Gly Ala Gln Glu
   610 615 620
   Gln Thr Pro Ser Gly Ala Ala Pro Ser Ser Pro Pro Pro Ile Ala Pro
   625 630 635 640
   Leu Arg Gly Met Arg Pro Gly Ser Arg Ala Arg Ser Ser Lys Gly Ser
   645 650 655
   Leu Ser Val Pro Lys Gly Gln Leu Pro Glu Val Gly Glu Glu Pro Ala
   660 665 670
   Glu Arg Pro Leu Asp Asp Arg Ala Glu Pro Glu Pro Pro Ala Ala Leu
   675 680 685
   Ser Pro Pro Lys Leu Ala Leu Asp Lys Pro Ser Trp Gly Ser Trp Ala
   690 695 700
   Ser Glu Val Ala Ser Val Glu Ala Arg Leu Ile Gly Pro Ile Ser Gly
   705 710 715 720
   Pro Lys Gly Gln Ile Val Glu Pro Glu Tyr Arg Gly Ile Val Pro Ser
   725 730 735
   Arg Gly Thr Thr Thr Ala Ala Ser Met Ala Ser Gly Thr Val Val Ser
   740 745 750
   Val Gly Arg Arg Ala Lys Gly Lys Glu Pro Glu Arg Ala Gln Ser Ala
   755 760 765
   Ser Ser Ser Ser Glu Pro Ala Ser Gly Gly Ser Arg Gly Glu Glu Leu
   770 775 780
<210> 9
   <211> 811
   <212> PRT
   <213> Mycovirus MoCV3
<400> 9
   Met Ala Met Gly Met Thr Gly Gly Gln Leu Ala Ser Lys Leu Arg Ile
   1 5 10 15
   Asp Gln Gly Leu Gly Leu Pro Asp Ala Phe Leu Lys Gln Ser Gly Arg
   20 25 30
   Ala Gly Glu Ala Asp Ser Gly Ala Asp Gly Glu Trp Ala Ala Phe Gly
   35 40 45
   Ile His Ser Gly Ala Pro Pro Asn Ala Ala His Pro His Val Val Pro
       50                  55                  60
   Ala Ala Leu Asp Ala Ser Ser Ser Ala Gly Arg Val Asp Thr Leu Arg
   65                  70                  75                  80
   Pro Leu Val Gly Asp Val Ala Tyr Ser Leu Phe Leu Arg Leu Gly Glu
                   85                  90                  95
   Thr Asp Tyr Asp Val Thr Lys Asp Glu Glu Ala Ser Pro Met Asp Val
               100                 105                 110
   Ser His Ser Val Ile Cys Ser Tyr Ala Leu Glu Ile Asp Gly Arg Thr
           115                 120                 125
   Ala Leu Thr Arg Ala Asp Val Thr Ser His Cys Ala Val Tyr Pro Pro
       130                 135                 140
   Met Ala Lys Arg Gly Ser Ala Thr Pro Val Ser Val Thr Gly Ala Leu
   145                 150                 155                 160
   Thr Thr Ser Arg Arg Thr Ser Ala Ala Ala Ala Cys Glu Gly Leu Ala
                   165                 170                 175
   Met His Ala Gly Ser Gly Asn Asn Ala Asp Val Ser Leu Gly Val Ala
               180                 185                 190
   Arg Glu Phe Met Tyr Asp Arg Ala Arg His Gln Glu Asn Gly Leu Glu
           195                 200                 205
   Ser Val Phe Val Arg Met Trp Leu Val His Leu Ser Val Leu Ser Arg
       210                 215                 220
   Arg Pro Val Thr Gln Val Val Asp Pro Ala Met Leu Ala Ala Arg Phe
   225                 230                 235                 240
   Val Asn Ile Leu Ala Pro Ala Glu Ser Asp Ala Ala Arg Ala Met Arg
                   245                 250                 255
   Gly Val Arg Ile Asn Ala Arg Gly Leu Ser Asp Thr Ala Leu Ala Leu
               260                 265                 270
   Leu Val Leu Gly Cys Ser Asp Thr Ser Gln Ala Ala Gly Leu His Tyr
           275                 280                 285
   Arg Ala Arg Arg Tyr Lys Phe Thr Arg Ser Ala Leu Ser Met Tyr Gly
   290 295 300
   Met Gln Gly Arg Ile Arg Leu Ala Val Ala Leu Asp Arg Ala Glu Val
   305 310 315 320
   Thr Gly Leu Ala Ile Val Ser Leu Ala Glu Arg Tyr Gly Ala Glu Ala
   325 330 335
   Ala Cys Gly Ala Gly Leu Gln Thr Ala Leu Met Met Tyr Gly Val Asn
   340 345 350
   Asp Ser Gly Arg Tyr Val Arg Leu Lys Cys Pro Glu Pro Glu Leu His
   355 360 365
   Asp Asp Val Ala Thr Thr Ala Gly Ile Arg Ala Leu Ser Val Arg Ser
   370 375 380
   Tyr Ser Asp Leu Ser Asp Asn Arg Leu Leu Ser Leu Ser Leu Phe Val
   385 390 395 400
   Gly Arg Ala Trp Arg Gln Ala Ala Gly His Leu Leu Arg Ser Ser Thr
   405 410 415
   Met Gln Thr Thr Thr Ala Asp Ile Asp Ala Val Val Asn Thr Leu Leu
   420 425 430
   Pro Ser Gln Gly Ala Leu Ile Lys Ala Val Gly Cys Ala His Ala Arg
   435 440 445
   Ala Met Gly Trp Val Ala Pro Leu Val Asp Thr Val Ser Tyr Val Glu
   450 455 460
   Gln Asn Tyr Arg Leu Leu Trp Glu Glu Arg Gly Val Val His Cys Leu
   465 470 475 480
   Ala Leu Gly Leu Arg Val Pro Asn Ser Val Leu Glu Glu Ala Thr Ala
   485 490 495
   Val Ile Asp Val Pro Tyr Pro Pro Ala Leu Ser Pro Ser Asp Asp Pro
   500 505 510
   Leu Ser Ala Lys Pro Arg Ala Asn Ser Leu Ala Thr Leu Gly Leu Val
   515 520 525
   Glu Ser Leu Leu Ser Ser Ser Gly Glu Ser Leu Cys Gly Ser Ala Arg
   530 535 540
   Ala Arg Gly Arg Arg Gln Ala Gly Leu Val Ala Val Pro Ala Gln Val
   545                 550                 555                 560
   Val Ala Leu Ala Gly His Arg Val Gln Phe Thr Leu Leu Ser Val Ala
                   565                 570                 575
   Ser Gly Val Ala Val Lys Val Glu Glu Leu Pro Thr Arg Pro Leu Leu
               580                 585                 590
   Ala Glu Pro Leu Gln Thr Gly Leu Gln Ala Val Glu Tyr Val Gln Ala
           595                 600                 605
   Pro Trp Ala Pro Ala Gln Asp Ala Pro Glu Pro Ala Pro Pro Val Glu
       610                 615                 620
   Phe Arg Asp Thr Leu Asp Gln Phe Leu His Ala Tyr Arg Pro Ser Arg
   625                 630                 635                 640
   Ser Val Leu Glu Gln Pro Arg Val Glu Val Thr Pro Arg Met Ser Ser
                   645                 650                 655
   Gly Ser Asp Val Leu Asn Val Met Leu Pro Glu Gly Thr Pro Ala Ser
               660                 665                 670
   Leu Arg Glu Ser Ile Ile Gly Gly Thr Leu Thr Pro Val Ser Gly Ala
           675                 680                 685
   Gly Val Gly Gly Cys Ala Glu Ala Ile Ala Asn Ser Leu Gln Ala Gln
       690                 695                 700
   His Gly Val Thr Val Ser Ala Gly Glu Ile Glu Lys Ala Met Gln Gly
   705                 710                 715                 720
   Asp Ser Gly Gly Thr Ser Leu Gln Val Glu Ala Met Ala Gly Ala Leu
                   725                 730                 735
   Ala Lys Phe Gly Asp Tyr Arg Leu Val Leu Leu Asp Glu His Ser Gln
               740                 745                 750
   Gly Val Ser Leu Lys Ala Gly Ala Ser Gly Ser Lys Pro Val Thr Ile
           755                 760                 765
   His Arg Asn Gly Ser Ser Tyr Asn Ala Leu Gly Arg Gly Pro Gly Arg
       770                 775                 780
   Gly Ile Arg Val Arg Leu Arg Gly Gly Gly Pro Pro Ala Arg Glu Ala
   785                 790                 795                 800
   Gln Pro Gln Leu Arg Arg Leu Ser Arg Ser Ser
                   805                 810
<210> 10
   <211> 666
   <212> PRT
   <213> Mycovirus MoCV3
<400> 10
   Met Asp Ala Lys Gln Ser Gly Ala Gly Ala Arg Val Ser Glu Trp Trp
   1               5                   10                  15
   Pro Asp Arg Gln Val Ile Ser Arg Glu Ala Leu Arg Gln Arg Thr Ser
               20                  25                  30
   Arg Thr Leu Gly Ala Met Gly Gly Ala Val Gly Val Ser Asp Thr Pro
           35                  40                  45
   Arg Ser Phe Phe Gly Gly Gly Glu Val Pro Thr Glu Arg Leu Arg Arg
       50                  55                  60
   Gly Leu Pro Ser Glu Val Ala Gly Met Phe Thr Ala Glu Gln Leu Ser
   65                  70                  75                  80
   Glu Met Leu Asp Met Ser Ala Glu Gln Arg Val Lys Ala Leu Ala Asp
                   85                  90                  95
   Ala Trp Glu Arg Leu Ala Thr Ala Glu Ala Ser Thr Gly Pro Thr Ser
               100                 105                 110
   Ser Gly Leu Gln Ala Pro Ala Arg Arg Ala Pro Arg Ser Val Ser Asp
           115                 120                 125
   Tyr Asn Thr Thr Gln Trp Val Ala Ala Ala Ala Ser Asp Gly Ile Arg
       130                 135                 140
   Val Thr Pro Thr Leu Thr Ala Ala Ala Arg Ala Val Ala Pro Ala Leu
   145                 150                 155                 160
   Asn Arg Ala Val Val Pro Gly Gly Cys Gln Pro His Ala Ser Tyr Gly
                   165                 170                 175
   Arg Ala Thr Val Ala Thr Gly Thr Gly Asp Val Ala Pro Pro Ser Ala
               180                 185                 190
   Arg Gln Thr Ala Val Val Gln Ala Ile Gly Glu Ser Val Pro Thr Glu
           195                 200                 205
   Pro Val Asp Ala Glu Val Lys Glu Gly Thr Pro Ser Pro Ala Ala Pro
       210                 215                 220
   Pro Arg Leu Leu Cys Gly Thr Ala Thr Leu Leu Ala Leu Pro Gln Val
   225                 230                 235                 240
   Cys Cys Gly Ser Ala Leu Asp His Gly Cys Ala Ala Ala Pro Cys Ala
                   245                 250                 255
   Val Gly Gln Val Ser Gly Ala Ala Ala Ala Val Ala Leu Ala Ser Arg
               260                 265                 270
   Pro Val Val Glu Pro Asp Ala Asp Ala Ser Ile Met Gly Glu Arg Trp
           275                 280                 285
   Lys Val Met Ser Thr Leu Ala Ala Arg Leu Gly Pro Ile Ala Gln Cys
       290                 295                 300
   His Phe Gly Ser Cys Arg Gly Val Cys Ala Cys Thr His Arg His Pro
   305                 310                 315                 320
   Leu Ala Arg Ala Leu Met Gly Ile Gly Val Gly Thr Glu Glu Leu Arg
                   325                 330                 335
   Gly Ala Arg Val His Leu Thr Thr Pro Gln Ala Ser Arg Ser Val Ala
               340                 345                 350
   Ser Val Leu Leu Glu Lys Val Gly Gly Gly Ser Arg Val Glu Arg Leu
           355                 360                 365
   Ala Val Glu Phe Val Lys Ala Val Asp Gly Pro Val Leu Thr Ser Leu
       370                 375                 380
   Val Val Ala Ser Arg Asp Gly Lys Ala Asp Ser Leu Leu Thr Leu Val
   385                 390                 395                 400
   Ala Glu Ala Met Ala Gln Gly Ser Leu Ala Ala Pro Arg Thr Met Val
                   405                 410                 415
   Thr Phe Gly Arg Gly Asp Gly Gly Gly Pro Asp Leu Asp Pro Asn Lys
               420                 425                 430
   Leu Tyr Asp Ala Cys Val Tyr Ser Gly Glu Arg Gly His Lys Leu Gly
           435                 440                 445
   Val Leu Ser Arg Ala Val Trp Leu Glu Ser Asp Ala Asp Val His Trp
       450                 455                 460
   Met Pro His Ser Asn Ser Leu Lys Val Thr Leu Gln Cys Trp Asn Arg
   465                 470                 475                 480
   Arg Gly Asp Cys Lys Gly Val Ser Ala Val Ile Gly Pro Thr Thr Trp
                   485                 490                 495
   Arg Pro His Val Gly Asn Ser Ser Trp Glu Thr Leu Gly Ser Asp Val
               500                 505                 510
   Tyr Arg Asp Trp Phe Gly Leu Val Val Ala Arg Arg Gly Gly Gly Asp
           515                 520                 525
   Thr Thr Val Arg His Gln Leu Ser Tyr Asn Pro Gly Met Glu Ala Asp
       530                 535                 540
   Gln Leu Gly Gly Leu Leu Ala His Ser Gly Cys Gly Val Tyr Val Ser
   545                 550                 555                 560
   Gly Ala Asp Asp Ala Ala Thr Val Ala Ala Val His Pro Ser Leu Gln
                   565                 570                 575
   Val Thr Tyr Thr His Ile Thr Leu Gly Ser Pro Thr Ala Ser Lys Leu
               580                 585                 590
   Val Gln Ala Ala Thr Ala Leu Asp Glu Phe Val Arg Arg Gly Gly Arg
           595                 600                 605
   Ile Ala His Glu Ala Ala Gly Thr Ala Asp Glu Ala Val Ile Thr Ala
       610                 615                 620
   Arg Asp Ala Gly Gly Arg Arg Gln Gly Arg Cys Thr Lys Ala Lys Ala
   625                 630                 635                 640
   Thr Ala Arg Ser Leu Gly Pro Ile Asp Asp Gly Ala Ser Gln Asp Gln
                   645                 650                 655
   Ala Pro Thr Arg Pro Arg Gly Gly Ala Leu
               660                 665
<210> 11
   <211> 34
   <212> DNA
   <213> Artificial
<220>
   <223> Synthetic DNA
<400> 11
   gccccggggc aaaaaagaga ataaagcttt ctcc 34
<210> 12
   <211> 34
   <212> DNA
   <213> Artificial
<220>
   <223> Synthetic DNA
<400> 12
   gttctagagg tacttacacc tcacagcgta agaa 34
<210> 13
   <211> 34
   <212> DNA
   <213> Artificial
<220>
   <223> Synthetic DNA
<400> 13
   gcgagctcgc aaaaaagaga ataaagcatt ccct 34
<210> 14
   <211> 34
   <212> DNA
   <213> Artificial
<220>
   <223> Synthetic DNA
<400> 14
   gtgttaacgg tacttacgtt gtcacgtaag aagt 34
<210> 15
   <211> 34
   <212> DNA
   <213> Artificial
<220>
   <223> Synthetic DNA
<400> 15
   gcgtcgacgc aaaaaagaga ataaagcttt ctcc 34
<210> 16
   <211> 34
   <212> DNA
   <213> Artificial
<220>
   <223> Synthetic DNA
<400> 16
   gttctagagg tacttgttgg gaccctacgt ccga 34
<210> 17
   <211> 34
   <212> DNA
   <213> Artificial
<220>
   <223> Synthetic DNA
<400> 17
   gcgtcgacgc aaaaaagaga ataaagcttt ctcc 34
<210> 18
   <211> 34
   <212> DNA
   <213> Artificial
<220>
   <223> Synthetic DNA
<400> 18
   gttctagagg tacttgttga agccccatgc tcaa 34
<210> 19
   <211> 34
   <212> DNA
   <213> Artificial
<220>
   <223> Synthetic DNA
<400> 19
   gccccggggc aaaaaagaga ataaagcatt ctcc 34
<210> 20
   <211> 34
   <212> DNA
   <213> Artificial
<220>
   <223> Synthetic DNA
<400> 20
   gttctagagg tacttacgtc atcacgtaag aagt 34

## Description

### Technical Field

The present invention relates to a novel mycovirus that suppresses the infectivity of a plant-pathogenic fungus against a plant. The present invention also relates to a plant-pathogenic fungus infected by the mycovirus, a plant disease control agent comprising the mycovirus, a method for controlling a plant disease using the mycovirus, and a method for attenuating a plant-pathogenic fungus using the mycovirus.

### Background Art

Plant diseases are caused by, for example, environmental factors such as weather or soil, infectious factors such as viruses, bacteria, or fungi (filamentous fungi), physiological disorders, or combinations of these factors. Still, a large number of plant diseases are disincentive to the production of food, flowers, flowering trees, timbers, etc. and mostly have a great economic impact.

Of these factors causing plant diseases, fungi are one of the most important causative factors. Approximately 80% of the plant diseases are allegedly caused by the fungi.

For example, rice blast disease is one of the most important plant diseases that occur all over the world. The pathogenic microbe of this disease is a rice blast disease fungus (scientific name: "*Magnaporthe oryzae*"), which is a mold (filamentous fungus). A temperature around 25 degrees C is appropriate for the growth, sporulation, or infection of *Magnaporthe oryzae,* while this fungus prefers a wet environment. Weather factors such as summer-time low temperature, heavy rain, or lack of sunlight therefore cause an outbreak of the fungus, which in turn brings about the poor harvest or reduced quality of rice, dealing a great blow to the economy.

There exist a large number of other plant diseases, such as sheath blight disease, rust disease, powdery mildew, anthrax, stem rot, downy mildew, and gray mold, which are caused by fungi and have a great economic impact. In this regard, attempts are still being made on the development of novel agricultural chemicals, breeding, etc. (for the control of rice blast disease, see, for example, Patent Literatures 1 and 2).

Hereinafter, mycoviruses, which are an item related to the present invention, will be described.

The mycoviruses refer to viruses that infect fungi. Among the mycoviruses, mycoviruses having double-stranded RNA genomes have been reported. The majority of these mycoviruses latently infect host fungi and rarely influence the traits of the hosts.

The mycoviruses having double-stranded RNA genomes are currently classified into five families including *Partitiviridae* (scientific name), *Totiviridae* (scientific name), and *Chrysoviridae* (scientific name). The *Partitiviridae* viruses have two linear double-stranded RNAs of almost the same size in their virions and have a total gene size of 4 to 6 kbp. The *Totiviridae* viruses have one linear double-stranded RNA of 4 to 7 kbp in their virions. In addition, a chestnut blight fungus (scientific name: *"Cryphonectria parasitica*") has been found to contain an endogenous virus having a double-stranded RNA of 9 to 13 kbp (hypovirus, etc.).

The *Chrysoviridae* viruses have spherical virus-like particles and have four double-stranded RNA segments. These viruses are known to have regions encoding RNA-dependent RNA polymerase (RdRP), as in the *Partitiviridae* and *Totiviridae* viruses. For example, *Helminthosporium victoriae* 145S virus (Hv145SV), *Penicillium chrysogenum* virus (PcV), and *Agaricus bisporus* virus 1 (AbV1) are known as viruses belonging to the family *Chrysoviridae* (see, for example, Non Patent Literature 1).

In recent years, methods involving attenuating pathogenic microbes of particular plant diseases with mycoviruses or the like and using the attenuated microbes to control the diseases have been studied, some of which have been put in actual use. There have been disclosed, for example, a method involving attenuating *Cryphonectria parasitica* using the full-length cDNA of a virus-derived double-stranded RNA that suppresses the toxicity of the fungus and applying the attenuated fungus to the control of chestnut blight (see, for example, Non Patent Literature 1) and a method involving discovering a double-stranded RNA virus that suppresses a violet root rot fungus (scientific name: *Helicobasidium mompa*) and using an attenuated strain of *Helicobasidium mompa* endogenously containing the double-stranded RNA to control violet root rot (see, for example, Non Patent Literature 2 and Patent Literature 3).

Alternatively, Patent Literature 4 discloses a mycovirus that infects *Magnaporthe oryzae* to reduce the infectivity of *Magnaporthe oryzae,* and an attenuated strain of a plant-pathogenic fungus infected by the mycovirus. Reportedly, use of the mycovirus disclosed in Patent Literature 4 can attenuate a plant-pathogenic fungus and can provide novel means of controlling a plant disease.

### Citation List

### Patent Literature

Patent Literature 1: JP Patent Publication (Kokai) No. 2004-143045 A (2004)
Patent Literature 2: JP Patent Publication (Kokai) No. 2003-250370 A (2003)
Patent Literature 3: JP Patent Publication (Kokai) No. 2001-78752 A (2001)
Patent Literature 4: International Publication No. WO 2009/093409

### Non Patent Literature

Non Patent Literature 1: C. M. Fauquet, Mary Ann Mayo, J. Maniloff, U. Desselberger, L. A. Ball, "Virus Taxonomy: Classification and Nomenclature of Viruses; Eighth Report Of The International Committee On Taxonomy Of Viruses", Elsevier Academic Press: p. 591-595 Non Patent Literature 2: Gil H. Choi and Donald L. Nuss, "Hypovirulence of chestnut blight fungus conferred by an infectious viral cDNA." Science. 1992 Aug 7; 257 (5071): 800-3 Non Patent Literature 3: H. Osaki et al, "Detection of Double-Stranded RNA Virus from a Strain of the Violet Root Rot Fungus Helicobasidium mompa Tanaka" Virus Genes 25: 2, 139-145, 2002

### Summary of Invention

### Technical Problem

Although Patent Literature 4 described above discloses a mycovirus that attenuates a plant-pathogenic fungus, use of the mycovirus or the attenuated strain of a plant-pathogenic fungus infected by the mycovirus fails to produce a sufficient control rate. Thus, the achievement of a higher control rate has been demanded. Accordingly, an object of the present invention is to provide a mycovirus having a higher control effect on a plant-pathogenic fungus, a plant-pathogenic fungus infected by the mycovirus, a plant disease control agent comprising the mycovirus and/or the plant-pathogenic fungus, a method for controlling a plant disease using the mycovirus and/or the plant-pathogenic fungus, and a method for attenuating a plant-pathogenic fungus.

### Solution to Problem

The present invention has attained the object and encompasses the followings:
(1) A mycovirus having 5 types of double-stranded RNAs, wherein the 5 types of double-stranded RNAs are polynucleotides comprising the nucleotide sequences represented by SEQ ID NOs: 1 to 5, respectively.
(2) A plant-pathogenic fungus which is a host infected by a mycovirus according to (1), wherein the host is *Magnaporthe oryzae.*
(3) A plant disease control agent comprising a mycovirus according to (1).
(4) A method for controlling a plant-pathogenic fungus, comprising a step of contacting a plant disease control agent according to (3) with a plant.
(5) A method for attenuating a plant-pathogenic fungus, comprising the step of allowing a mycovirus according to (1) to infect the plant-pathogenic fungus.
(6) The method for attenuating a plant-pathogenic fungus according to (5), wherein the plant-pathogenic fungus is *Magnaporthe oryzae.*

### Advantageous Effects of Invention

The mycovirus according to the present invention is superior in control rate against a plant-pathogenic fungus to conventional mycoviruses. Thus, use of the mycovirus according to the present invention and a plant-pathogenic fungus infected by the mycovirus can control a plant disease with better efficiency than ever.

### Brief Description of Drawings

[Figure 1] Figure 1 is a photograph showing results of observing the colony of a *Magnaporthe oryzae* S-0412-II 2a strain or a *Magnaporthe oryzae* S-0412-II 1a strain in a PDA medium.
[Figure 2] Figure 2 is a photograph showing results of observing the colony of a virus-cured strain of the *Magnaporthe oryzae* S-0412-II 2a strain or the *Magnaporthe oryzae* S-0412-II 1a strain in a PDA medium.
[Figure 3] Figure 3(A) is a characteristic diagram showing the amounts of hyphae produced by the *Magnaporthe oryzae* S-0412-II 2a strain and its virus-cured strain. Figure 3(B) is a characteristic diagram showing the amounts of hyphae produced by the *Magnaporthe oryzae* S-0412-II 1a strain and its virus-cured strain.
[Figure 4] Figure 4 is a graph showing the number of lesions resulting from the spray inoculation of the *Magnaporthe oryzae* S-0412-II 2a strain.
[Figure 5] Figure 5 is an electron microscopic photograph of MoCV3 particles.
[Figure 6] Figure 6 is a diagram showing the constitution of a plasmid obtained by the PCR amplification of a full-length MoCV3 cDNA clone dsRNA1, dsRNA2, dsRNA3, dsRNA4, or dsRNA5 followed by subcloning into pUC 19.
[Figure 7] Figure 7 is a diagram showing the constitution of pRSA313, pRSA314, or pRSA315 used for constructing a shuttle vector.
[Figure 8] Figure 8 is a diagram showing the constitution of pRSA316 or pRSA317 used for constructing a shuttle vector.
[Figure 9] Figure 9(a) is a diagram showing results of performing Western blotting using anti-MoCV3 antiserum for a fraction obtained by the fractionation of reconstructed MoCV3 through sucrose concentration-gradient centrifugation. Figure 9(b) is a diagram showing results of observing MoCV3 virus-like particles under electron microscope.

### Description of Embodiments

Hereinafter, the present invention will be described in detail. The mycovirus according to the present invention has 5 types of double-stranded RNAs. The mycovirus according to the present invention has the function of infecting a rice blast disease fungus (scientific name: *"Magnaporthe oryzae*") and attenuating a plant-pathogenic fungus such as *Magnaporthe oryzae.*

Specifically, the mycovirus according to the present invention has 5 types of double-stranded RNAs comprising the nucleotide sequences represented by SEQ ID NOs: 1 to 5. In this context, all of the nucleotide sequences of SEQ ID NOs: 1 to 5 in the Sequence Listing are described as DNA sequences. All of these sequences also encompass RNA sequences (thymine is replaced with uracil).

The mycovirus according to the present invention can be preserved in an endogenous form in *Magnaporthe oryzae.* One example of the mycovirus according to the present invention can include *Magnaporthe oryzae* chrysovirus 3 (MoCV3) identified and designated by the present inventors. This MoCV3 has 5 types of double-stranded RNAs consisting of the nucleotide sequences represented by SEQ ID NOs: 1 to 5 and is preserved in an endogenous form in *Magnaporthe oryzae.*

A *Magnaporthe oryzae* S-0412-II 2a strain endogenously containing this MoCV3 has been demonstrated to fall under the refusal of deposition in National Institute of Technology and Evaluation (NITE), Patent Microorganisms Depositary on the ground of endogenously containing a virus. This strain is also being stored in Laboratory of Plant Pathology, Tokyo University of Agriculture and Technology and can be furnished to third parties on the condition of compliance with laws and regulations. In addition, this strain is in the process of deposition with American Type Culture Collection.

The place where this fungal strain has been originated is Vietnam. Of the double-stranded RNAs, the nucleotide sequence represented by SEQ ID NO: 1 contains a region encoding the conserved motif of RNA-dependent RNA polymerase (RdRP), and this region has a nucleotide sequence homology to *Chrysoviridae* viruses. Thus, this mycovirus is presumably a novel viral species classified into the family *Chrysoviridae.*

This MoCV3 is very similar to MoCV1 disclosed in International Publication No. WO 2009/093409, but differs in its excellent ability to attenuate *Magnaporthe oryzae,* compared with MoCV1. The 4 types of double-stranded RNAs represented by SEQ ID NOs: 1 to 4 out of the 5 types of double-stranded RNAs in MoCV3 exhibited 80.5%, 74.5%, 71.3%, and 72.5% homologies, respectively, to 4 types of double-stranded RNAs in MoCV1. Specifically, a mycovirus comprising 4 types of double-stranded RNAs comprising nucleotide sequences having 81%, 75%, 72%, and 73% or higher homologies to the 4 types of double-stranded RNAs represented by SEQ ID NOs: 1 to 4, respectively, is incorporated as a novel mycovirus in the technical scope of the present disclosure.

Alternatively, the mycovirus according to the present disclosure may be a mycovirus that has 5 types of double-stranded RNAs having, for example, 85% or higher, preferably 90% or higher, more preferably 95% or higher, most preferably 97% or higher homologies, to the nucleotide sequences represented by SEQ ID NOs: 1 to 5, respectively, and can attenuate a plant-pathogenic fungus such as *Magnaporthe oryzae.* In this context, the "homology" refers to being completely identical between nucleotide sequences. "Similarity" refers to being completely identical between nucleotide sequences as a result of replacing adenine with guanine (or vice versa) or thymine (uracil) with cytosine (or vice versa).

The mycovirus according to the present disclosure is not limited to this MoCV3 and also encompasses a mycovirus artificially or naturally mutated from MoCV3. Specifically, the mycovirus according to the present disclosure may be a variant that comprises a nucleotide sequence derived from any of the nucleotide sequences represented by SEQ ID NOs: 1 to 5 by the substitution, deletion, addition, or insertion of one or several (e.g., 2 to 100, preferably 2 to 50, more preferably 2 to 25, most preferably 2 to 10) bases and can attenuate a plant-pathogenic fungus such as *Magnaporthe oryzae.*

The nucleotide sequences represented by SEQ ID NOs: 1 to 5 contain coding regions of their respective proteins. The amino acid sequences of putative proteins encoded by the coding regions contained in the nucleotide sequences of SEQ ID NOs: 1 to 5 are shown in SEQ ID NOs: 6 to 10, respectively.

The mycovirus according to the present invention has the effect of suppressing, for example, the growth of a plant-pathogenic fungus. Thus, for example, a predetermined plant-pathogenic fungus can be allowed to endogenously contain this mycovirus through its infection or the like to thereby prepare an attenuated strain of the plant-pathogenic fungus.

For example, a plant disease control agent containing the mycovirus according to the present invention and/or the attenuated strain of the plant-pathogenic fungus thus prepared may be added (e.g., distributed or applied) to a plant (rice, etc.) to thereby control its plant disease. In addition, the mycovirus according to the present invention has features as shown below. Mycoviruses have heretofore been considered to be transmitted vertically from the cells of host fungi to cells of other fungi through hyphal fusion and not to exist outside the cells of the host fungi at any stage of their life cycles. By contrast, the study of the present inventors demonstrated that the mycovirus according to the present invention can exist even extracellularly.

Thus, the mycovirus according to the present invention can infect a host fungus from outside its cell in a manner independent of hyphal fusion. The mycovirus according to the present invention can therefore cause transmission even between a wide range of fungal species or fungal strains differing in mating type and can infect a host fungus with high efficiency. This is likely to achieve simple and highly efficient attenuation of a plant-pathogenic fungus or control of a plant disease.

Since the mycovirus according to the present invention can exist even extracellularly, for example, the host fungus is cultured in a liquid medium and the mycovirus can be recovered from a culture supernatant thereof to thereby produce the mycovirus simply and in a relatively large amount.

For example, the mycovirus according to the present invention endogenously contained in a predetermined strain of *Magnaporthe oryzae* can be separated and recovered from the *Magnaporthe oryzae* strain by an approach known in the art. In this context, the *Magnaporthe oryzae* strain endogenously containing the mycovirus according to the present invention can be cultured in the same medium under the same culture conditions as in usual *Magnaporthe oryzae.*

### <Gene, nucleic acid, protein, etc. according to the present invention>

The present inventors have conducted sequencing analysis on one mycovirus according to the present invention to obtain its full-length nucleotide sequence (SEQ ID NOs: 1 to 5). Thus, the present disclosure encompasses all of genes of the mycovirus, nucleic acids having their nucleotide sequences or a portion thereof, proteins encoded by these nucleotide sequences, etc.

The present disclosure also encompasses all of nucleic acids having the whole or a portion of these nucleotide sequences. The nucleic acids may be double-stranded or single-stranded and encompass all of DNAs, cDNAs, RNAs, etc.

The present disclosure also encompasses, for example, cDNAs having all or any of the sequences of SEQ ID NOs: 1 to 5, the sequences of particular sites having predetermined functions in (any of) the sequences of SEQ ID NOs: 1 to 5, or nucleotide sequences equivalent thereto, and recombinant vectors (plasmids, viruses, etc.) with incorporated nucleotide sequences equivalent thereto.

The results of sequencing analysis demonstrated that: a partial site of the sequence of SEQ ID NO: 1 contains the conserved motif of RNA-dependent RNA polymerase (RdRP); and a partial site of the sequence of SEQ ID NO: 4 has a homology to the double-stranded RNA fragment of a La France disease virus. Thus, for example, nucleic acids or recombinant vectors having at least these partial sites of the sequences as particular sites having predetermined functions may be prepared and used according to the purpose or use.

The nucleic acids (or genes) according to the present disclosure encompass a wide range of nucleic acids having homologies to the nucleotide sequences described above, for example, nucleic acids that hybridize under stringent conditions to nucleic acids consisting of nucleotide sequences complementary to the nucleotide sequences described above and have the effect of suppressing *Magnaporthe oryzae.* In this context, the stringent conditions can be determined by a technique known in the art with reference to, for example, the Tm values of double-stranded nucleic acids.

The present disclosure also encompasses all proteins encoded by the mycovirus genes and nucleic acids described above. The amino acid sequences of the proteins according to the present disclosure are shown in SEQ ID NOs: 6 to 10.

SEQ ID NO: 6 represents an amino acid sequence for an open reading frame in the nucleotide sequence described in SEQ ID NO: 1. SEQ ID NO: 7 represents an amino acid sequence for an open reading frame in the nucleotide sequence described in SEQ ID NO: 2. SEQ ID NO: 8 represents an amino acid sequence for an open reading frame in the nucleotide sequence described in SEQ ID NO: 3. SEQ ID NO: 9 represents an amino acid sequence for an open reading frame in the nucleotide sequence described in SEQ ID NO: 4. SEQ ID NO: 10 represents an amino acid sequence for an open reading frame in the nucleotide sequence described in SEQ ID NO: 5.

The proteins according to the present disclosure encompass all of proteins having any of the amino acid sequences of SEQ ID NOs: 6 to 10 as well as proteins that have homologies thereto and maintain their functions.

For example, the nucleic acids described above may be incorporated into recombinant vectors and forcedly expressed by hosts to thereby prepare their proteins in large amounts. Any host known in the art, such as *E. coli* strains, yeast strains, or cultured cells, may be available. Considering that, for example, the mycovirus infects a fungus and yeast strains are highly proliferative and are relatively conveniently used, the yeast strains may be the optimum hosts. Likewise, any recombinant vector known in the art may be available.

For example, the 5 types of genes can be coexpressed using a plurality of vectors each containing any of the nucleotide sequence fragments of SEQ ID NOs: 1 to 5 to thereby reconstruct the mycovirus. In this case as well, any host known in the art and any recombinant vector known in the art are available. Yeast strains are the optimum hosts because a plurality of vectors can be introduced thereinto simultaneously. In this context, the 5 types of nucleic acid fragments represented by SEQ ID NOs: 1 to 5 can be obtained by RT-PCR with the double-stranded RNAs (dsRNAs) extracted from the mycovirus as templates. Alternatively, the 5 types of nucleic acid fragments represented by SEQ ID NOs: 1 to 5 may be totally synthesized on the basis of their sequence information. In other words, those skilled in the art can produce the mycovirus according to the present invention using the recombinant vectors even if the mycovirus itself and/or the *Magnaporthe oryzae* S-0412-II 2a strain endogenously containing the mycovirus is not deposited with a depositary institution.

### <Attenuated strain of plant-pathogenic fungus>

The attenuated strain of a plant-pathogenic fungus according to the present invention encompasses all of strains endogenously containing the mycovirus according to the present invention. Specifically, the attenuated strain of a plant-pathogenic fungus according to the present invention encompasses, for example, both of a fungal strain (e.g., *Magnaporthe oryzae*) already endogenously containing the mycovirus and a strain of a plant-pathogenic fungus infected by the mycovirus.

For example, a conventional method involving allowing the mycovirus to infect a host fungus through hyphal fusion can be used as means of allowing the mycovirus to infect a plant-pathogenic fungus. Alternatively, the mycovirus according to the present invention can exist even extracellularly, as mentioned above, and may therefore be allowed to infect a host fungus, for example, directly from outside its cell.

Examples of this attenuated strain of a plant-pathogenic fungus include the S-0412-II 2a strain described above. This fungal strain is a strain of *Magnaporthe oryzae* infected by the mycovirus according to the present invention. Thus, the S-0412-II 2a strain is basically similar in morphological properties, cultural properties, sporulation, and physiological and chemotaxonomic properties to *Magnaporthe oryzae* known in the art except that: the strain grows slower than usual *Magnaporthe oryzae*; its hyphae grow non-concentrically; the strain exhibits nonuniform pigmentation; the abnormal development of aerial hyphae is seen; and sector formation or lysis is observed.

### <Plant disease control agent>

The plant disease control agent according to the present invention encompasses all of control agents containing at least the mycovirus according to the present invention. Alternatively, the plant disease control agent according to the present invention may contain both of the mycovirus and the attenuated strain of a plant-pathogenic fungus and may contain an additional ingredient.

The additional ingredient that may be contained therein includes, for example, predetermined carriers, binders, thickeners, fixing agents, preservatives and fungicides, solvents, stabilizers, antioxidants, UV protective agents, crystal deposition inhibitors, antifoaming agents, physical property-improving agents, and coloring agents. The plant disease control agent according to the present invention may also contain additional agricultural chemical ingredient(s), for example, a miticide, a nematicide, a germicide, an antivirus agent, an attractant, a herbicide, a plant growth regulator, and/or a synergist.

For example, either of a solid carrier or a liquid carrier, or both, can be used as the carriers. Examples of the solid carrier include: animal- or plant-derived powders such as starch, active carbon, soybean flour, wheat flour, wood flour, fish flour, and powdered milk; and mineral powders such as talc, kaolin, bentonite, zeolite, diatomaceous earth, white carbon, clay, alumina, calcium carbonate, potassium chloride, and ammonium sulfate. Examples of the liquid carrier include: water; alcohols such as isopropyl alcohol and ethylene glycol; ketones such as cyclohexanone and methyl ethyl ketone; ethers such as propylene glycol monomethyl ether and diethylene glycol mono-n-butyl ether; aliphatic hydrocarbons such as kerosine and light oil; aromatic hydrocarbons such as xylene, trimethylbenzene, tetramethylbenzene, methylnaphthalene, and solvent naphtha; amides such as N-methyl-2-pyrrolidone; esters such as glycerin ester of fatty acid; and plant oils such as soybean oil and rapeseed oil.

Examples of the binders, the thickeners, and the fixing agents include starch, dextrin, cellulose, methylcellulose, ethylcellulose, carboxymethylcellulose, hydroxyethylcellulose, hydroxypropylcellulose, hydroxypropylmethylcellulose, carboxymethyl starch, pullulan, sodium alginate, ammonium alginate, alginic acid propylene glycol ester, guar gum, locust bean gum, gum arabic, xanthan gum, gelatin, casein, polyvinyl alcohol, polyethylene oxide, polyethylene glycol, ethylene-propylene block polymer, sodium polyacrylate, and polyvinylpyrrolidone.

The dosage form of the control agent of the present invention is not particularly limited. For example, forms such as emulsions, suspensions, dusts, granules, tablets, wettable powders, water-soluble powders, liquid formulations, flowable formulations, water-dispersible granules, aerosols, pastes, oil solutions, or concentrated emulsions can be applied thereto.

### <Method for producing mycovirus capable of suppressing plant-pathogenic fungus>

The method for producing a mycovirus capable of suppressing a plant-pathogenic fungus according to the present invention encompasses all of methods comprising at least the procedures of culturing a mycovirus-containing plant-pathogenic fungus in a liquid medium or the like and recovering the mycovirus from a culture supernatant thereof.

As mentioned above, the mycovirus according to the present invention can exist even outside the cell of a host fungus. Thus, for example, a plant-pathogenic fungus infected by the mycovirus is cultured in a liquid medium or the like, and the fungus body can be separated by centrifugation, followed by separation and recovery of the virus from a culture supernatant thereof to thereby recover the virus simply and in a relatively large amount.

However, the mycovirus according to the present invention is not narrowly limited to the mycovirus obtained by this production method. Specifically, the present invention encompasses, for example, a wide range of mycoviruses obtained by separation and recovery from *Magnaporthe oryzae* endogenously containing the mycovirus.

The method for producing a mycovirus capable of suppressing a plant-pathogenic fungus according to the present invention also includes a method involving, as mentioned above, allowing a host cell to coexpress the 5 types of genes using a plurality of vectors each containing any of the nucleic acid fragments of SEQ ID NOs: 1 to 5 and recovering the mycovirus thus reconstructed in the host cell. A yeast cell can be used as the host cell. The vectors to which the 5 types of genes are incorporated are not particularly limited as long as the vectors can cause the expression of the incorporated genes in the host cell. Any vector can be used. The nucleic acid fragments to be incorporated to the vectors can be totally synthesized on the basis of the nucleotide sequences represented by SEQ ID NOs: 1 to 5.

### <Method for attenuating plant-pathogenic fungus>

As mentioned above, for example, the mycovirus according to the present invention can be allowed to infect a particular plant-pathogenic fungus to thereby suppress, for example, the growth of the host fungus and attenuate the fungus. The same method as above can be adopted as means of allowing the mycovirus to infect a fungus.

### <Method for controlling plant disease>

The method for controlling a plant disease according to the present invention encompasses all of methods comprising at least the step of adding the rice blast disease control agent to a particular plant (rice, etc.).

Examples of means of adding the control agent to a plant include a method involving applying the control agent to the front side or back side of each leaf, a method involving attaching the control agent to the front side or back side of each leaf using a predetermined carrier or the like, and a method involving distributing or supplying the control agent to leaves.

The amount of the control agent applied or distributed can be appropriately selected according to various conditions such as the concentration of the active ingredient, the form of the preparation, the type of the target disease or crop, the degree of damage caused by the disease, the place where the control agent is used, the method for use, the timing of use, and the amount and type of a drug, a fertilizer, or the like used together or in combination with the control agent.

For example, a solution containing conidia of an attenuated strain of *Magnaporthe oryzae* adjusted to 1 × 10³ to 1 × 10¹⁰ conidia/mL may be sprayed or supplied in an amount of 1 to 1,000 mL per leaf, or 1 × 10³ to 1 x 10¹⁰ conidia of the attenuated strain of *Magnaporthe oryzae* per mm² of each leaf may be applied or attached to the front side or back side of the leaf, to thereby suppress the plant disease.

Alternatively, a virus solution containing a moderate dilution (approximately 10 to 100 times the undiluted form) of MoCV3 present in the culture supernatant or a moderate dilution of MoCV3 virion components extracted from the fungus body of the *Magnaporthe oryzae* S-0412-II 2a strain can be distributed directly to rice leaves to thereby achieve a control effect on *Magnaporthe oryzae* or a method for controlling *Magnaporthe oryzae* by a curing effect. As an actual control rate, MoCV3 used as a viral spray produced results of 63.6 or higher control rates, which are better than the control rate 56.8 or higher of MoCV1 disclosed in International Publication No. WO 2009/093409.

The present invention may be applicable to every plant disease mainly caused by a fungus. Examples of the plant disease to which the preset invention is applicable include, but not limited to, the followings:

Examples of the plant diseases in *Poaceae* plants include rice blast disease (causative fungus "*Magnaporthe oryzae*"), rice leaf spot (causative fungus *"Cochliobolus miyabeanus*"), sheath blight disease (causative fungus "*Thanatephorus cucumeris"*)*,* bakanae disease (causative fungus "*Gibberella fujikuroi"*)*,* damping off (causative fungi "*Fusarium* spp.", *"Rhizopus* spp.", "*Pythium* spp.", and "*Trichoderma viride"*)*,* rice false smut (causative fungus "*Claviceps virens"*)*,* wheat head blight disease (causative fungi "*Gibberella zeae*", "*Fusarium avenaceum*", "*Fusarium culmorum",* and "*Monographella nivale*"), snow mold (causative fungi "*Pythium* spp.", "*Typhula* spp.", *"Monographella nivalis*"*,* and "*Myriosclerotinia borealis*"), loose smut (causative fungus "*Ustilago nuda*"), bunt (causative fungus "*Tilletia controversa*"), eyespot disease (causative fungus "*Pseudocercosporella herpotrichoides"*)*,* leaf blotch (causative fungus "*Septoria tritici"*)*,* glume blotch (causative fungus "*Phaeosphaeria nodorum*"), and powdery mildew (causative fungus "*Blumeria graminis*").

Other examples of the plant diseases include: melanose (causative fungus "*Diaporthe citri*"), melanose-like blemish (causative fungi "*Diaporthe medusaea*" and "*Alternaria citri"*)*,* common scab (causative fungus *"Elsinoe fawcettii*"), brown rot (causative fungus "*Phytophthora citrophthora*"), green mold (causative fungus *"Penicillium digitatum*"), and blue mold (causative fungus *"Penicillium italicum*") in citrus; monilia disease (causative fungus "*Monilinia mali*"), scab (causative fungus "*Venturia inaequalis*"), Alternaria blotch (causative fungus "*Alternaria mali*"), melanose (causative fungus "*Mycosphaerella pomi*"), sooty blotch (causative fungus *"Gloeodes pomigena"*)*,* flyspeck (causative fungus "*Zygophiala jamaicensis*"), ring rot (causative fungus "*Botryosphaeria berengeriana*"), brown spot (causative fungus "*Diplocarpon mali*"), rust (causative fungus "*Gymnosporangium yamadae"*)*,* and Valsa canker (causative fungus "*Valsa ceratosperma*") in apple; scab (causative fungus "*Venturia nashicola*"), rust (causative fungus "*Gymnosporangium asiaticum*"), ring rot (causative fungus "*Botryosphaeria berengeriana*"), and blight (causative fungus *"Phomopsis fukushii*") in Japanese pear; leaf curl (causative fungus "*Taphrina deformans*"), brown rot (causative fungi "*Monilinia fructicola*" and "*Monilinia fructigena*"), scab (causative fungus "*Cladosporium carpophilum*"), and Phomopsis rot (causative fungus "*Phomopsis* sp.") in peach; brown rot (causative fungus "*Monilinia fructicola*" and "Monilinia fructigena") and young fruit stem rot (causative fungus "*Monilinia kusanoi*") in yellow peach; scab (causative fungus "*Cladosporium carpophilum*") in Japanese apricot; anthracnose (causative fungus *"Elsinoe ampelina*"), ripe rot (causative fungi *"Colletotrichum acutatum*" and "*Glomerella cingulata*"), brown spot (causative fungus "*Pseudocercospora vitis"*)*,* and dead arm (causative fungus *"Phomopsis viticola*") in grape; angular leaf spot (causative fungus "*Cercospora kak*i") and circular leaf spot (causative fungus "*Mycosphaerella nawae*") in persimmon; gray blight (causative fungi "*Pestalotiopsis longiseta*" and "*Pestalotiopsis theae*"), brown round spot (causative fungi "*Pseudocercospora ocellata*" and "*Cercospora chaae*"), blister blight (causative fungus "*Exobasidium vexans*"), and net blister blight (causative fungus "*Exobasidium reticulatum*") in tea; gummy stem blight (causative fungus "*Mycosphaerella melonis"),* dead arm (causative fungus "*Fusarium oxysporum*"), scab (causative fungus "*Cladosporium cucumerinum*"), and brown spot (causative fungus "*Corynespora cassiicola*") in gourd; leaf mold (causative fungus "*Fulvia fulva*") and ring rot (causative fungus "*Alternaria solani*") in tomato; Phomopsis blight (causative fungus *"Phomopsis vexans*") and leaf mold (causative fungus "*Mycovellosiella nattrassii*") in eggplant; white rust (causative fungus *"Albugo macrospora*") and white spot (causative fungi "*Cercosporella brassicae*" and "*Pseudocercosporella capsellae*") in *Brassicaceae* vegetables; neck rot (causative fungus "*Botrytis allii*") in onion; leaf spot (causative fungus "*Mycosphaerella fragariae*") in strawberry; early blight (causative fungus "*Alternaria solani*") in potato; Phytophthora rot (causative fungus "*Phytophthora sojae*") and purple seed stain (causative fungus "*Cercospora kikuchii"*) in soybean; Phytophthora rot (causative fungus "*Phytophthora vignae*") in adzuki bean; brown spot (causative fungus "*Mycosphaerella arachidis*") in peanut; brown spot (causative fungus "*Cercospora beticola*") and foliage blight (causative fungus "*Thanatephorus cucumeris"*) in sugar beet; Curvularia leaf blight (causative fungi "*Curvularia* spp."), dollar spot disease (causative fungus "*Sclerotinia homoeocarpa*"), and Helminthosporium leaf blight (causative fungi *"Cochliobolus* spp.") in turf grass; scab (causative fungus "*Diplocarpon rosae*") in rose; white rust (causative fungus "*Puccinia horiana*") in chrysanthemum; downy mildew (causative fungi "*Peronospora* spp.", "*Pseudoperonospora* spp.", "*Plasmopara* spp.", and "*Bremia* spp."), Phytophthora rot (causative fungus "*Phytophthora* spp."), powdery mildew (causative fungi "*Erysiphe* spp.", "*Blumeria* spp.", "*Sphaerotheca* spp.", "*Podosphaera* spp.", "*Phyllactinia* spp.", "*Uncinula* spp.", and "*Oidiopsis* spp."), rust disease (causative fungi "*Puccinia* spp.", "*Uromyces* spp.", and "*Physopella* spp."), black spot (causative fungi "Alternaria spp."), gray mold (causative fungus "*Botrytis cinerea*"), stem rot (causative fungus "*Sclerotinia sclerotiorum"),* white root rot (causative fungus "*Rosellinia necatrix*"), violet root rot (causative fungus "*Helicobasidium mompa*"), and southern blight (causative fungus "Sclerotium rolfsii") in various crops; and other various soilborne diseases (causative fungi "*Fusarium* spp.", "*Rhizoctonia* spp.", "*Pythium* spp.", "*Aphanomyces* spp.", "*Phoma* spp.", "*Verticillium* spp.", "*Plasmodiophora* brassicae", etc.).

### Examples

Hereinafter, the present invention will be described in more detail with reference to Examples. However, the technical scope of the present invention is not limited by Examples below.

### [Example 1]

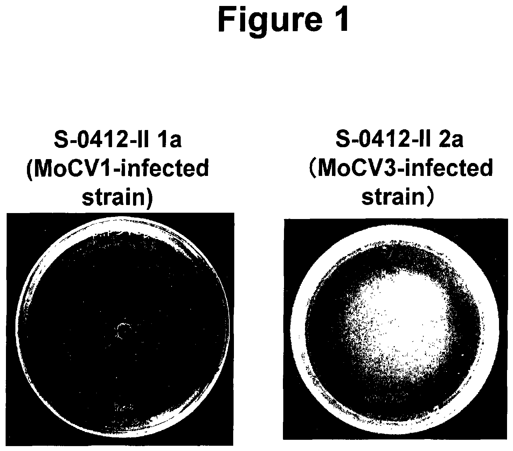

In this Example, a *Magnaporthe oryzae* S-0412-II 2a strain endogenously containing a novel mycovirus was isolated and identified according to the approach disclosed in International Publication No. WO 2009/093409. Specifically, the *Magnaporthe oryzae* S-0412-II 2a strain isolated and identified in this Example and a *Magnaporthe oryzae* S-0412-II 1a strain endogenously containing a mycovirus identified in International Publication No. WO 2009/093409 were separately cultured, and their colonies were observed. A PDA medium was used in the culture of these *Magnaporthe oryzae* strains. The results are shown in Figure 1.

As shown in Figure 1, the *Magnaporthe oryzae* S-0412-II 2a strain identified in this Example exhibited white albino flora, indicating the observable inhibition of growth. In addition, no conidiation was observed. Unlike the *Magnaporthe oryzae* S-0412-II 1a strain, the *Magnaporthe oryzae* S-0412-II 2a strain formed a characteristic colony in such a manner that: pigmentation (melanization) did not occur on a PDA medium; and abnormal and poor growth such as the growth inhibition and wetting of aerial hyphae was exhibited.

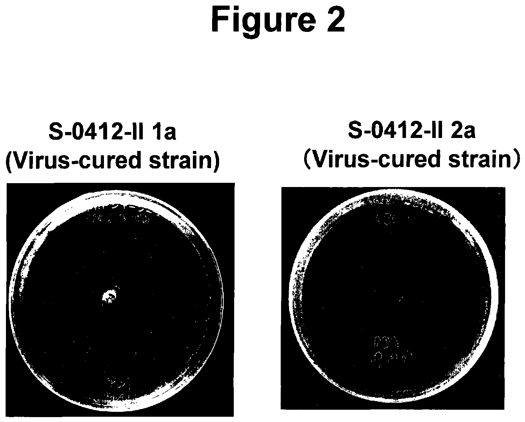

Virus-cured strains of these *Magnaporthe oryzae* strains S-0412-II 2a and S-0412-II 1a were prepared, and their colonies were similarly observed. The results are shown in Figure 2. In this context, the method for curing each virus was performed according to the approach disclosed in International Publication No. WO 2009/093409. As shown in Figure 2, both of the fungal strains formed colonies similar to those of usual *Magnaporthe oryzae,* as a result of virus cure.

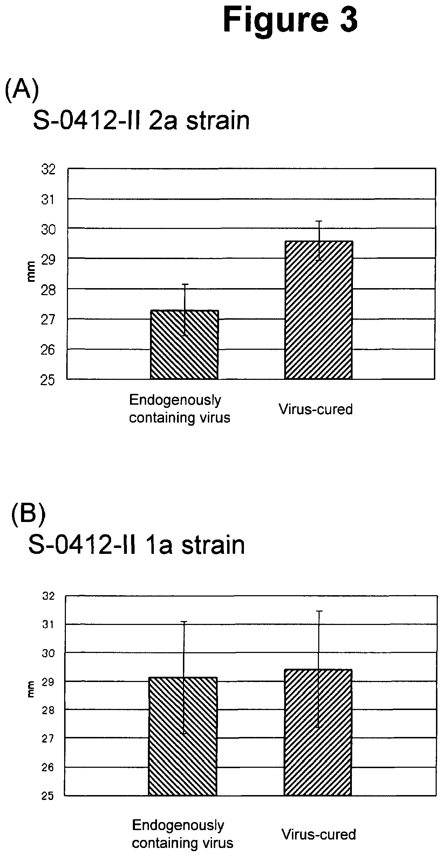

A growth rate was compared between the *Magnaporthe oryzae* S-0412-II 2a strain and the *Magnaporthe oryzae* S-0412-II 1a strain and their virus-cured strains. The method for this comparison involved measuring the distance from the center of the medium to the tip of a hypha. In this context, growth conditions for all the fungal strains were set to a cycle involving a light period of 12 hours and a dark period of 12 hours and a culture period of 9 days using a PDA medium. The results are shown in Figures 3A and 3B. Figure 3A shows the measurement results about the *Magnaporthe oryzae* S-0412-II 2a strain and its virus-cured strain. Figure 3B shows the measurement results about the *Magnaporthe oryzae* S-0412-II 1a strain and its virus-cured strain. As shown in Figures 3A and 3B, the *Magnaporthe oryzae* S-0412-II 1a strain did not exhibit a significant difference in growth rate even after virus cure. By contrast, the *Magnaporthe oryzae* S-0412-II 2a strain exhibited a significant difference in growth rate as a result of virus cure, demonstrating that the virus-infected strain had a reduced growth rate.

These results of Figures 1 and 2 clearly showed that the growth of both the fungal strains was suppressed owing to their endogenous mycoviruses. As shown in Figure 3, the mycovirus endogenously contained in the *Magnaporthe oryzae* S-0412-II 2a strain in this Example was shown to have the more potent ability to suppress the growth of *Magnaporthe oryzae*, compared with the mycovirus (MoCV1) endogenously contained in the *Magnaporthe oryzae* S-0412-II 1a strain.

The mycovirus endogenously contained in the *Magnaporthe oryzae* S-0412-II 2a strain isolated and identified in this Example was designated as MoCV3. It was concluded that this MoCV3 was a strong growth-suppressing factor (attenuating factor) against *Magnaporthe oryzae.*

### [Example 2]

In this Example, MoCV3 endogenously contained in the *Magnaporthe oryzae* S-0412-II 2a strain isolated and identified in Example 1 was examined for its existence outside the fungus body.

Of fungal strains with which the bands of 5 segments of endogenous double-stranded RNAs were detected at 2.8 to 3.6 kb in Example 1, 3 strains were separately transplanted and cultured in a liquid medium. Then, each culture solution was centrifuged, and a culture supernatant thereof was recovered. Next, the obtained culture supernatant was electrophoresed on 1% agarose gel at 20 V for 18 hours and stained with ethidium bromide.

As a result, double-stranded RNA bands were also detected, at the same positions as in the endogenous double-stranded RNAs, in the culture supernatants of these fungal strains. This result shows that the mycovirus according to the present invention exists not only within the fungus body of *Magnaporthe oryzae* but outside the fungus body.

### [Example 3]

In this Example, the mycovirus MoCV3 existing outside the fungus body was examined for its ability to infect a normal fungal strain (fungal strain carrying no mycovirus).

First, the *Magnaporthe oryzae* S-0412-II 2a strain was transplanted to a liquid medium and cultured for 4 weeks. Then, the culture solution was centrifuged, and a culture supernatant thereof was recovered. This culture supernatant was electrophoresed on 1% agarose gel by the same procedures as in Example 2. As a result, double-stranded RNA bands were successfully confirmed. The obtained culture supernatant was filter-sterilized through a 0.22-µL filter.

Next, 50 ml of a YG medium was placed in 100-ml Kolben, to which a normal fungal strain (fungal strain carrying no mycovirus) of *Magnaporthe oryzae* was then inoculated and cultured for 3 days. Then, 500 µL of the obtained culture supernatant was added thereto, and the growth of the fungus body was observed.

As a result, at observation day 3, a fungal strain non-supplemented with the culture supernatant exhibited normal growth with the tips of hyphae growing upright, whereas the hyphae of the fungal strain supplemented with the culture supernatant were entangled with their tips not much growing.

This result suggests that the mycovirus present in the culture supernatant infected the normal fungal strain of *Magnaporthe oryzae* to suppress its growth. Specifically, the results of this experiment show that the mycovirus existing outside the fungus body has the ability to infect a normal fungal strain.

### [Example 4]

In this Example, the number of conidia of the *Magnaporthe oryzae* strain infected by the mycovirus was measured.

The *Magnaporthe oryzae* S-0412-II 2a strain was transplanted to a YG plate and cultured for 2 weeks. Then, the fungus body was extracted and collected using a cork ball having a diameter of 4 mm. Next, 5% glycerol was placed in a 1.5-ml tube, to which the collected fungus body was added and mixed for 5 minutes for suspension. Then, the number of conidia present in the suspension was counted using a counting chamber.

As a result, the number of conidia was 33 × 10⁴ conidia/mL for the normal fungal strain (control; fungal strain carrying no mycovirus) of *Magnaporthe oryzae*, but was 1 × 10⁴ or less conidia/mL for the *Magnaporthe oryzae* S-0412-II 2a strain.

This result shows that the mycovirus according to the present invention suppresses the conidiation of a host fungus. This means that MoCV3 can suppress the proliferation of a plant-pathogenic fungus and can also suppress its transmission, the spread of infection, or the like.

### [Example 5]

In this Example, double-stranded RNAs extracted from the *Magnaporthe oryzae* S-0412-II 2a strain were sequenced. The extraction and purification of double-stranded RNAs from the *Magnaporthe oryzae* S-0412-II 2a strain, the synthesis of cDNAs with the obtained double-stranded RNAs as templates, and the subsequent analysis of both terminal sequences by 5' RACE were performed according to the approach disclosed in International Publication No. WO 2009/093409.

The results clearly showed that MoCV3 contained 5 types of double-stranded RNAs. The nucleotide sequences of these 5 types of double-stranded RNAs are shown in SEQ ID NOs: 1 to 5. Since the double-stranded RNAs were sequenced after reverse transcription into cDNAs, "uracil" is replaced with "thymine" in the Sequence Listing.

As a result of analyzing the obtained nucleotide sequences, the RNA sequence represented by SEQ ID NO: 1 contained the conserved motif of RNA-dependent RNA polymerase (RdRP) that is found in *Totiviridae* viruses and their related viruses, etc. The RNA sequence represented by SEQ ID NO: 4 contained a region having a homology to the double-stranded RNA fragment L3 of a La France disease virus.

This result shows that the 5 types of double-stranded RNAs according to the present invention offer genetic information on the novel mycovirus.

### [Example 6]

In this Example, the *Magnaporthe oryzae* S-0412-II 2a strain was studied by the spray inoculation method for its effectiveness in controlling a plant-pathogenic fungus.

The *Magnaporthe oryzae* S-0412-II 2a strain was inoculated to an oatmeal medium plate and cultured indoors at 25 degrees C. When conidia were insufficiently formed 15 days after the inoculation, approximately 2 mL of sterilized distilled water was poured to the plate, and the surface of the medium was brushed to remove aerial hyphae. This plate was placed under black light for 3 days to induce conidiation. Approximately 1 mL of sterilized distilled water was poured thereto, and the surface of the medium was brushed again to recover conidia together with aerial hyphae. In this way, a solution containing conidia was obtained. When conidia were sufficiently formed 15 days after the inoculation, approximately 2 mL of sterilized distilled water was poured to the plate, and the surface of the medium was brushed to recover conidia together with aerial hyphae. In this way, a solution containing conidia was obtained. A mycovirus-completely cured strain of *Magnaporthe oryzae* was prepared as a control by the same procedures as in Example 1. A solution containing conidia was obtained from the fungal strain by the same procedures as above.

Next, each solution containing conidia was filtered through Kimwipe or gauze to adjust the conidia concentration to 2 × 10⁶ conidia/mL. 0.02% (v/v) Tween 20 was added thereto to prepare a conidium suspension.

Next, the conidium suspension was sprayed evenly over rice seedlings (breed appropriately selected in consideration of a genotype of true resistance to rice blast disease and the race of the fungus; 2 to 3 weeks after seeding) using a nozzle. The plants were left standing for 24 hours in an inoculation chamber set to 26 degrees C and 100% relative humidity. Then, the pots were transferred to a greenhouse and cultured for 7 days after the spray inoculation while the temperature of the room was kept at 23 to 30 degrees C. Seven days after the spray inoculation, the number of susceptible lesions that became 3 to 4 mm per given area of each leaf was measured.

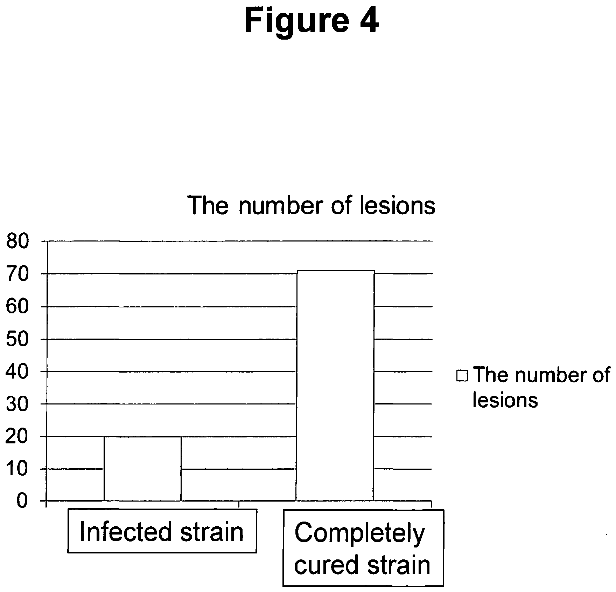

The results are shown in Figure 4. Figure 4 is a graph showing the number of lesions in the leaf inoculated with *Magnaporthe oryzae.* In the diagram, the ordinate denotes the number of lesions in the leaf inoculated with *Magnaporthe oryzae.* In the diagram, "Mixed infected strain" represents the number of lesions resulting from the spraying of the conidium suspension prepared from *Magnaporthe oryzae* infected by the mycovirus according to the present invention; and "Completely cured strain" represents the number of lesions (control) resulting from the spraying of the conidium suspension prepared from the mycovirus-completely cured strain of *Magnaporthe oryzae.*

As shown in Figure 4, the spraying of the conidium suspension prepared from *Magnaporthe oryzae* infected by the mycovirus according to the present invention remarkably decreased the number of lesions, compared with the control. This result shows that the *Magnaporthe oryzae* S-0412-II 2a strain is effective in controlling a plant-pathogenic fungus.

### [Example 7]

In this Example, the *Magnaporthe oryzae* S-0412-II 2a strain was studied by the punch inoculation method for its effectiveness in controlling a plant-pathogenic fungus.

A conidium suspension was prepared by the same procedures as in Example 6 and attached to 3% plain agar film. The agar film was cut into approximately 2 mm square to prepare an agar section. The 4th leaf of each rice seedling cultivated in a greenhouse at 23 to 30 degrees C was punched using a punch for inoculation to form a wet wound. The agar section was placed on the wounded portion. The plant was left standing for 24 hours in an inoculation chamber set to 26 degrees C and 100% relative humidity. Then, the pot was transferred to a greenhouse and cultured for 14 days after the inoculation while the temperature of the room was kept at 23 to 30 degrees C. 14 days after the inoculation, the size of each lesion was measured.

As a result, the plant inoculated with the conidium suspension prepared from the *Magnaporthe oryzae* S-0412-II 2a strain had a remarkably small lesion, compared with the control. This result shows that the strain of the present invention is effective in controlling a plant-pathogenic fungus, as in Example 6.

### [Example 8]

In this Example, MoCV3 particles existing outside the fungus body were identified using an electron microscope. Prior thereto, MoCV3 virions were analyzed for their biochemical properties in the same way as the disclosed approach. As a result, the main component (coat protein) of a virus protein was confirmed by SDS-PAGE to have a molecular weight of approximately 70 kDa. Specifically, the presence of MoCV3 particles was successfully confirmed.

In electron microscopic observation, the *Magnaporthe oryzae* S-0412-II 2a strain was first cultured in a YG medium (0.5% yeast extract and 2% glucose). Virions were isolated from a culture supernatant thereof. The culture supernatant was centrifuged (10,000 × g, 5 min.), and the obtained supernatant was ultracentrifuged (100,000 × g, 30 min.) to obtain virion-containing precipitates. The precipitates were dissolved in a 0.05 M phosphate buffer (pH 7.0), then negatively stained with phosphotungstic acid or uranium acetate, and observed under electron microscope (magnification: × 20,000 to 40,000).

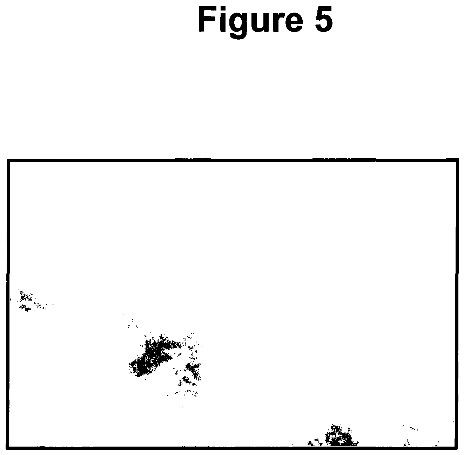

The results are shown in Figure 5. Figure 5 is an electron microscopic photograph showing the virions obtained from the culture supernatant of the *Magnaporthe oryzae* S-0412-II 2a strain. As shown in Figure 5, the virions of the mycovirus according to the present invention were successfully identified using an electron microscope. These virions were in a regular hexagon-like form of approximately 30 to 40 nm and included in envelope-like structures.

### [Example 9]

In this Example, isolated MoCV3 was distributed as a spray directly to rice leaves and studied for its control effect and curing effect on *Magnaporthe oryzae.*

Specifically, MoCV3 present in the culture supernatant was moderately diluted (approximately 10 to 100 times the undiluted form) to prepare a virus solution as a virus spray solution. Next, the diluted virus solution derived from the culture supernatant was sprayed to rice seedlings after a lapse of 2 weeks to 3 weeks from germination. Next, *Magnaporthe oryzae* was allowed to infect the plants. Then, a control rate in each test section was calculated. The control rate was calculated as Control rate = 100 - (Damage of treated section / Damage of untreated section) × 100 according to Guidelines on Assessment for the Designation of Specified Control Materials (Specified Agricultural Chemicals).

As a result, MoCV3 used as a viral spray produced results of 63.6 or higher control rates. The control rate of MoCV1 disclosed in International Publication No. WO 2009/093409 was calculated in the same way as above and was consequently 56.8. This result clearly showed that MoCV3 endogenously contained in the *Magnaporthe oryzae* S-0412-II 2a strain was superior in control effect on *Magnaporthe oryzae* or *Magnaporthe oryzae* control effect based on its curing effect to the conventionally known mycovirus.

### [Example 10]

In this Example, the reconstruction of MoCV3 virions in yeast cells was studied. Specifically, in order to reconstruct the MoCV3 virus in the cell of *Saccharomyces cerevisiae,* full-length cDNA clones were prepared with five dsRNA genomes carried by MoCV3 as templates, and ligated respectively to 5 types of shuttle vectors (see below) having marker genes different from one another. MoCV3 gene products were expressed in *Saccharomyces cerevisiae* cells to reconstruct MoCV3 virions.

### <Construction of shuttle vector for gene expression in yeast>

Backbone vectors pRS313, pRS314, pRS315, and pRS316 were purchased from National BioResource Project (http://yeast.lab.nig.ac.jp/nig/index.html). Backbone vectors pRS317, pRS412, pRS423, pRS424, pRS425, and pRS426 were purchased from ATCC (http://www.atcc.org/).

An alcohol dehydrogenase 1 (ADH1) cassette (ADH1 promoter + MCS + ADH1 terminator) was excised from pAUR123 (manufactured by Takara Bio Inc.) using BamHI and subcloned into pUC19. In order to construct a glyceraldehyde-3-phosphate dehydrogenase (TDH3) cassette, an amplification product containing half a multicloning site (MCS) added to the 3' end of the 600-bp upstream region of TDH3 and an amplification product containing the remaining half of MCS added to the 5' end of the 350-bp downstream region of TDH3 were first subcloned by PCR with the genomic DNA of a *Saccharomyces cerevisiae* strain W303-1A (Mat a ura3 leu2 his3 trp1 ade2 can1) as a template. The obtained two constructs were ligated to prepare a TDH3 cassette having the 600-bp TDH3 upstream region, MCS, and the 350-bp TDH3 downstream region in this order.

MCS was removed from each of the backbone vectors, and the ADH1 cassette or the TDH3 cassette was in turn inserted to the site. The vectors pRS313, pRS314, pRS315, pRS316, and pRS317 having the ADH1 inserts were designated as pRSA313, pRSA314, pRSA315, pRSA316, and pRSA317, respectively. The vectors pRS313, pRS314, pRS315, pRS316, pRS317, pRS423, pRS424, pRS425, and pRS426 having the TDH3 inserts were designated as pRST313, pRST314, pRST315, pRST316, pRST317, pRST423, pRST424, pRST425, and pRST426, respectively.

### <Preparation of shuttle vector construct for expression of MoCV3-derived gene (dsRNA1, dsRNA2, dsRNA3, dsRNA4, and dsRNA5)>

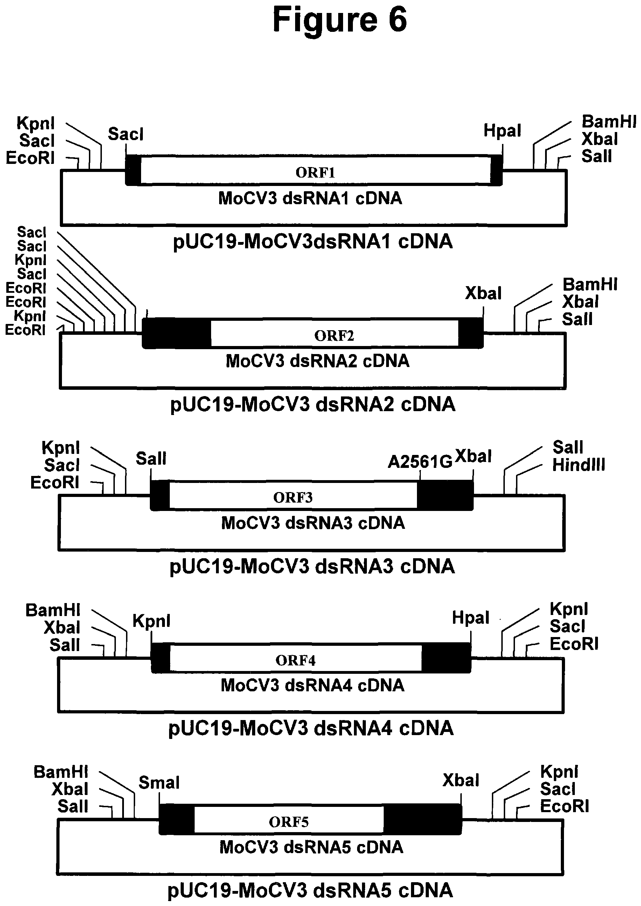

Full-length sequences were amplified by PCR with the full-length MoCV3 cDNA clones dsRNA1, dsRNA2, dsRNA3, dsRNA4, and dsRNA5 as templates. Each obtained DNA fragment was subcloned into pUC19 and then subjected to sequencing analysis to select accurate clones (Figure 6). The region of interest was excised from each obtained clone using restriction enzymes, then purified, and inserted to the shuttle vectors. Finally, the absence of variation in the inserted sequences and their flanking sequences was confirmed by sequencing.

### <Preparation of full-length MoCV3 cDNA>

### <dsRNA1>

A full-length DNA fragment was obtained by RT-PCR with the dsRNA1 segment separated by native PAGE or purified MoCV3 dsRNA as a template. 20 pmol each of dsRNA1-specific primers (MoCV3-cDNA-dsRNA1-5end-SmaI and MoCV3-cDNA-dsRNA1-3end-XbaI) was added to the template dsRNA (approximately 50 ng-100 ng) dissolved in distilled water, and the mixture was incubated at 98 degrees C for 5 minutes and then rapidly cooled on ice for 3 minutes. Then, 40 µl of the reaction system was incubated at 42 degrees C for 30 minutes and then at 70 degrees C for 15 minutes under conditions involving 1 × first strand synthesis buffer (manufactured by Invitrogen Corp.), 1 mM CH₃HgOH, 10 mM DDT, 1 mM dNTP mix, 40 U RNase OUT Recombinant (manufactured by Invitrogen Corp.), and 200 U Super Script III reverse transcriptase. Then, 5 U RNase H was added thereto. After incubation at 37 degrees C for 10 minutes and then at 70 degrees C for 15 minutes, the cDNA was purified and concentrated using QIAquick PCR Purification Kit (manufactured by Qiagen N.V.). This cDNA solution was used as a template in PCR using 2 U Pfu-x (manufactured by Greiner Bio-One International AG) and a buffer included therein, and phosphorylating primers (MoCV3-cDNA-dsRNA1-5end-SmaI: GCC CCG GGG CAA AAA AGA GAA TAA AGC TTT CTC C (SEQ ID NO: 11) and MoCV3-cDNA-dsRNA1-3end-XbaI: GTT CTA GAG GTA CTT ACA CCT CAC AGC GTA AGA A (SEQ ID NO: 12)). The reaction conditions were set to 95 degrees C for 2 minutes, then 30 cycles each involving 95 degrees C for 30 seconds, 55 degrees C for 30 seconds, and 68 degrees C for 3.5 minutes, followed by 68 degrees C for 7 minutes. After agarose gel electrophoresis, the cDNA amplification fragment of interest was extracted and purified from the gel and incorporated to a plasmid vector pUC19 DNA SmaI (manufactured by MBI Fermentas Inc.) using DNA Ligation kit Ver. 2.1 (manufactured by Takara Bio Inc.). The full-length sequence of the inserted fragment in a clone obtained by blue white selection was obtained by sequencing. A clone having a sequence completely identical to that of MoCV3 dsRNA1 was selected.

### <dsRNA2>

A clone having a sequence completely identical to that of MoCV13 dsRNA4 was obtained by the same approach as in the preparation of the full-length cDNA of dsRNA1 except that dsRNA2-specific primers (MoCV3-cDNA-dsRNA2-5end-SacI: GCG AGC TCG CAA AAA AGA GAA TAA AGC ATT CCC T (SEQ ID NO: 13) and MoCV3-cDNA-dsRNA2-3end-Hpa1: GTG TTA ACG GTA CTT ACG TTG TCA CGT AAG AAG T (SEQ ID NO: 14)) were used.

### <dsRNA3>

A clone having a sequence completely identical to that of MoCV13 dsRNA4 was obtained by the same approach as in the preparation of the full-length cDNA of dsRNA1 except that dsRNA3-specific primers (MoCV3-cDNA-dsRNA3-5end-Sal1: GCG TCG ACG CAA AAA AGA GAA TAA AGC TTT CTC C (SEQ ID NO: 15) and MoCV3-cDNA-dsRNA3-3end-Xba1: GTT CTA GAG GTA CTT GTT GGG ACC CTA CGT CCG A (SEQ ID NO: 16)) were used.

### <dsRNA4>

A clone having a sequence completely identical to that of MoCV13 dsRNA4 was obtained by the same approach as in the preparation of the full-length cDNA of dsRNA1 except that dsRNA4-specific primers (MoCV3-cDNA-dsRNA4-5end-Sal1: GCG TCG ACG CAA AAA AGA GAA TAA AGC TTT CTC C (SEQ ID NO: 17) and MoCV3-cDNA-dsRNA4-3end-Xba1: GTT CTA GAG GTA CTT GTT GAA GCC CCA TGC TCA A (SEQ ID NO: 18)) were used.

### <dsRNA5>

A clone having a sequence completely identical to that of MoCV13 dsRNA5 was obtained by the same approach as in the preparation of the full-length cDNA of dsRNA1 except that dsRNA5-specific primers (MoCV3-cDNA-dsRNA5-5end-Sma1: GCC CCG GGG CAA AAA AGA GAA TAA AGC ATT CTC C (SEQ ID NO: 19) and MoCV3-cDNA-dsRNA5-3end-Xba1: GTT CTA GAG GTA CTT ACG TCA TCA CGT AAG AAG T (SEQ ID NO: 20)) were used.

### <Transformation of yeast with plasmid DNA>

### <Lithium acetate method>

Transformation was performed by the lithium acetate method as follows: first, yeast was transplanted to 5 ml of a liquid medium and precultured at 30 degrees C for 10 to 16 hours. After the culture, absorbance (OD₆₀₀) was measured, and the yeast was transplanted to 50 ml of a liquid medium at OD₆₀₀ = 0.1 to 0.2. The yeast was cultured at 100 rpm at 30 degrees C until OD₆₀₀ = 0.8. The cells were recovered by centrifugation and then washed by suspension in distilled water. The precipitated cells were suspended in Sol A (0.1 M lithium acetate, 10 mM Tris-HCl pH 7.8, and 1 mM EDTA) and centrifuged. The precipitates were suspended by the addition of Sol A again to prepare a yeast solution. This solution was incubated at 30 degrees C for 50 minutes. Then, 10 µl of heat-treated ssDNA (1 mg/ml; derived from salmon testis), 50 µl of the yeast solution, 10 µl of each shuttle vector, 20 µl of Sol A, and 750 µl of 50% polyethylene glycerol were added in this order and then incubated at 30 degrees C for 30 minutes and then at 42 degrees C for 15 minutes. After centrifugation, distilled water was added to the precipitates. The mixture was applied to an SC plate agar medium (dropout medium was selected according to the selective marker in the shuttle vector) and cultured at 30 degrees C.

### <Spheroplast method>

Transformation was performed by the spheroplast method as follows: first, yeast cells were cultured and recovered in the same way as the lithium acetate method. The cells were suspended in a 1.2 M sorbitol solution and recovered by centrifugation (2500 rpm, 5 min.). The precipitated cells were suspended again in a 1.2 M sorbitol solution. Zymolyase (20T, 1/10 volume) was added thereto. The cells were incubated at 30 degrees C until the great majority thereof became spheroplasts. The cells were washed three times with a 1.2 M sorbitol solution and suspended in in an STC solution. 23 µul each plasmid solution, 1 µl of heat-treated ssDNA (1 mg/ml; derived from salmon testis), and 4 ml of a PEG solution were added to 100 µl of this spheroplast suspension and gently mixed. The mixture was left standing for 10 minutes and then centrifuged (1200 rpm, 5 min.) to precipitate the spheroplasts, which were then suspended in 150 µl of an SOS solution. After culture at 30 degrees C for 1 hour, the suspension was mixed with 5 ml of an SD sorbitol-low melting point agarose solution and applied to an SD sorbitol plate.

### <Electroporation method>

Transformation was performed by the electroporation method as follows: first, yeast cells were cultured and recovered in the same way as the lithium acetate method. The cells were recovered by centrifugation and then washed by suspension in distilled water. The cells were suspended in 12.5 ml of a LiAC/DTT/TE solution and left standing at room temperature for 1 hour. Then, the cells were precipitated by centrifugation (6,000 rpm, 5 min.). The precipitates were washed twice by suspension in 12.5 ml of ice-cold water. The cells were suspended in 5 ml of a 1 M sorbitol solution and centrifuged (6,000 rpm, 5 min.) to precipitate the cells. The precipitates were suspended in 50 µl of a 1 M sorbitol solution to prepare a cell suspension. 5 µl of each plasmid solution was added to 70 µl of the cell suspension and left standing on ice for 5 minutes. This solution was applied to a 0.2-cm cuvette and subjected to electroporation at 1.5 kV, 25 (µFD, and 200 ohms. Immediately thereafter, 1 ml of a 1 M sorbitol solution was added thereto, and the mixture was applied to a 1 M sorbitol plate medium.

### <Reconstruction of MoCV3 in yeast by introduction of MoCV3 dsRNA1, dsRNA2, dsRNA3, dsRNA4, and dsRNA5>

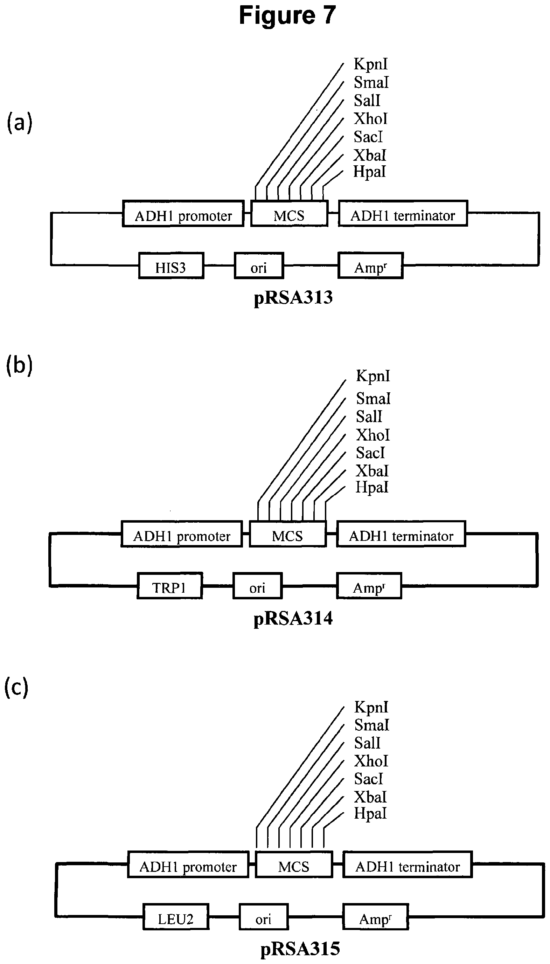

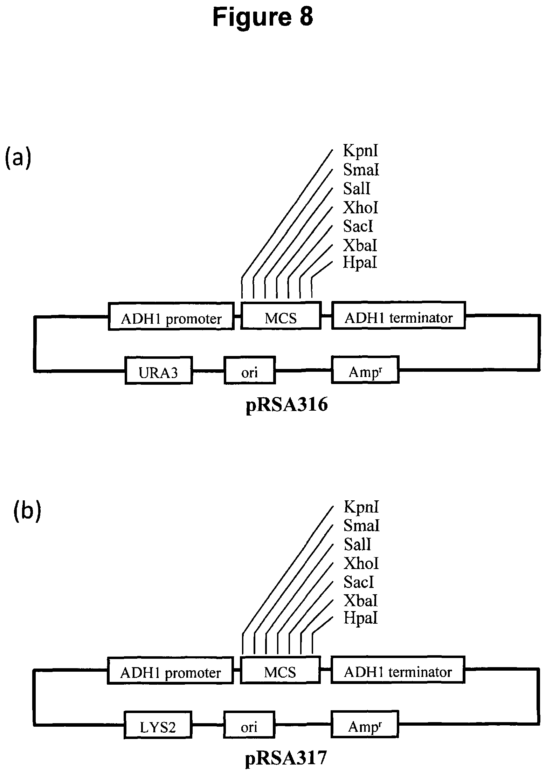

In order to prepare pRSAA313-dsRNA1, pRSA314-dsRNA2, pRSA315-dsRNA3, pRSA316-dsRNA4, and pRSA317-dsRNA5 for expression of MoCV3 dsRNA1 to dsRNA5, shuttle vectors were constructed in which the full-length cDNA clones of dsRNA1 to dsRNA5 prepared by the methods described above were ligated to the restriction enzyme sites of pRSA313, pRSA314, pRSA315, pRSA316, and pRSA317, respectively (Figures 7 and 8). For the ligation of the full-length cDNA of dsRNA1 to pRSA313, restriction enzyme sites SmaI and XbaI were used (Figure 7a). For the ligation of the full-length cDNA of dsRNA2 to pRSA314, restriction enzyme sites SacI and HpaI were used (Figure 7b). For the ligation of the full-length cDNA of dsRNA3 to pRSA315, restriction enzyme sites SalI and XbaI were used (Figure 7c). For the ligation of the full-length cDNA of dsRNA4 to pRSA316, restriction enzyme sites SalI and XbaI were used (Figure 8a). For the ligation of the full-length cDNA of dsRNA5 to pRSA317, restriction enzyme sites SmaI and XbaI were used (Figure 8b). The dsRNA1 to dsRNA5 gene expression shuttle vectors thus obtained by ligation were introduced to a *Saccharomyces cerevisiae* YPH499 strain (Mat a ura3 lys2 ade2 leu2 his3 trp1) by the lithium acetate method.

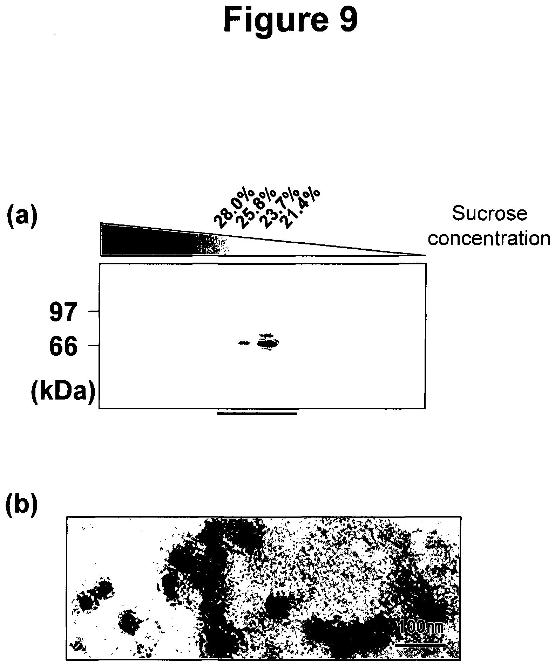

The obtained transformant colony was cultured in a microbial culture apparatus to attempt the purification of virus-like particles. The yeast cells were disrupted using a French press. Then, the homogenates were purified in the same way as the method for purifying MoCV3 virions and fractionated by sucrose concentration-gradient centrifugation. The obtained fraction was subjected to Western blotting using anti-MoCV3 antiserum (Figure 9a). As a result, a signal that appeared to be derived from the MoCV3 protein was detected around 24% sucrose concentration of the MoCV3 dsRNA1-5-introduced yeast sample. As a result of electron microscopic observation, MoCV3 virus-like particles were observed (Figure 9b).

## Claims

1. A mycovirus having 5 types of double-stranded RNAs, wherein the 5 types of double-stranded RNAs are polynucleotides comprising the nucleotide sequences represented by SEQ ID NOs: 1 to 5, respectively.

2. A plant-pathogenic fungus which is a host infected by a mycovirus according to claim 1, wherein the host is *Magnaporthe oryzae.*

3. A plant disease control agent comprising a mycovirus according to claim 1.

4. A method for controlling a plant-pathogenic fungus, comprising a step of contacting a plant disease control agent according to claim 3 with a plant.

5. A method for attenuating a plant-pathogenic fungus, comprising the step of allowing a mycovirus according to claim 1 to infect the plant-pathogenic fungus.

6. The method for attenuating a plant-pathogenic fungus according to claim 5, wherein the plant-pathogenic fungus is *Magnaporthe oryzae.*

## Patentansprüche

1. Mykovirus mit 5 Arten doppelsträngiger RNAs, worin die 5 Arten doppelsträn-giger RNAs Polynucleotide sind, die die Nucleotidsequenzen, die durch die Seq.-ID Nr. 1 bis 5 dargestellt sind, umfassen.

2. Pflanzenpathogener Pilz, der ein von einem Mykovirus nach Anspruch 1 infizierter Wirt ist, wobei der Wirt *Magnaporthe oryzae* ist.

3. Pflanzenerkrankungskontrollmittel, das ein Mykovirus nach Anspruch 1 umfasst.

4. Verfahren zur Kontrolle eines pflanzenpathogenen Pilzes, umfassend den Schritt des Kontaktierens eines Pflanzenerkrankungskontrollmittels nach Anspruch 3 mit einer Pflanze.

5. Verfahren zur Attenuation eines pflanzenpathogenen Pilzes, umfassend den Schritt, einem Mykovirus nach Anspruch 1 zu erlauben, den pflanzenpathogenen Pilz zu infizieren.

6. Verfahren zur Attenuation eines pflanzenpathogenen Pilzes nach Anspruch 5, worin der pflanzenpathogene Pilz *Magnaporthe oryzae* ist.

## Revendications

1. Mycovirus possédant 5 types d'ARN double brin, dans lequel les 5 types d'ARN double brin sont des polynucléotides comprenant les séquences de nucléotides représentées par SEQ ID NO: 1 à 5, respectivement.

2. Champignon pathogène pour une plante qui est un hôte infecté par un mycovirus selon la revendication 1, où l'hôte est *Magnaporthe oryzae.*

3. Agent de contrôle d'une maladie végétale comprenant un mycovirus selon la revendication 1.

4. Procédé pour contrôler un champignon pathogène pour une plante, comprenant une étape consistant à mettre en contact un agent de contrôle d'une maladie végétale selon la revendication 3 avec une plante.

5. Procédé pour atténuer un champignon pathogène pour une plante, comprenant l'étape consistant à permettre à un mycovirus selon la revendication 1 d'infecter le champignon pathogène pour une plante.

6. Procédé pour atténuer un champignon pathogène pour une plante selon la revendication 5, dans lequel le champignon pathogène pour une plante est *Magnaporthe oryzae.*
